# EUROPEAN PATENT APPLICATION

(11) **EP 4 477 652 A1**
(43) Date of publication of application: **18.12.2024**
(21) Application number: 23749331.7
(22) Date of filing: 07.02.2023
(51) Int. Cl.: C07D 403/12, A61K 31/53, A61P 3/06

(54) **POLYMORPH AS THYROID HORMONE RECEPTOR AGONISTS AND USE THEREOF**

(30) Priority: 07.02.2022 CN 202210120295; 04.01.2023 CN 202310008505
(71) Applicant: Xizang Haisco Pharmaceutical Co., Ltd., Lhoka, Tibet 856099 (CN)
(72) Inventor: FAN, Jiang, Chengdu, Sichuan 611130 (CN); GONG, Zheng, Chengdu, Sichuan 611130 (CN); ZHU, Fengfei, Chengdu, Sichuan 611130 (CN); FENG, Jianchuan, Chengdu, Sichuan 611130 (CN); LI, Yao, Chengdu, Sichuan 611130 (CN); DOU, Ying, Chengdu, Sichuan 611130 (CN)
(74) Representative: Calysta NV
(86) International application number: PCT/CN2023/000038
(87) International publication number: WO 2023/147752

(57) **Abstract**

Disclosed in the present invention are a polymorph of a compound of formula I (R)-2-(3,5-dichloro-4-((7-(methyl-d3)-1-oxo-2,5,6,7-tetrahydro-1H-cyclopenta[d]pyridazin-4-yl)oxy)phenyl)-3,5-dioxo-2,3,4,5-tetrahydro-1,2,4-triazine-6-carbonitrile, a preparation method, pharmaceutical composition and pharmaceutical use therefor.

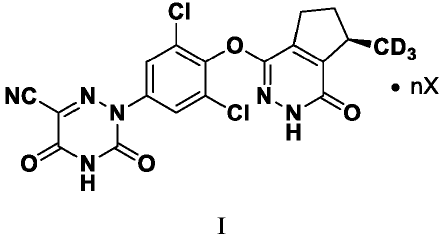

## Description

### Technical Field

The present invention belongs to the pharmaceutical field and in particular to a polymorph of a thyroid hormone receptor agonist, a preparation method therefor and use thereof.

### Background Art

Classical familial hypercholestemlemia (FH) is an autosomal (co) dominant genetic disorder, with the main clinical manifestation of a marked increase in serum low density lipoprotein-cholesterol (LDL-C) level, as well as skin/tendon xanthoma, and can lead to early cardiovascular disease. Common FHs are divided into heterozygous familial hypercholesterolemia (HeFH) and, more rarely, homozygous familial hypercholesterolemia (HoFH). According to statistics, the prevalence of HeFH is as high as 0.2%-0.48%. FH is usually caused by inactivating mutations in the LDL receptor (LDLR). In clinical use, statin medications are the drugs of first choice for treatment, not only to reduce LDL-C levels, but also to improve the prognosis of the patients with FH. Patients who do not respond well to statin therapy or have adverse reactions can be treated in combination with the cholesterol absorption inhibitor of ezetimibe. PCSK9 inhibitor may be additionally administered to patients who do not reach the standard cholesterol level after the treatments described above. Although there are corresponding treatments with respect to HeFH, many patients (up to 40% of HeFH patients) are unable to reach the standard level of cholesterol (LDL-C) after these treatments, and the accumulation of cholesterol in their bodies becomes a lifelong burden.

Thyroid hormone receptors are divided into two subtypes, α and β subtypes. Binding of thyroid hormone to the β subtype receptor can promote the cholesterol metabolism. As a result, several thyroid hormone analogs or thyromimetics that selectively agonise the β subtype receptor have been developed in recent years. The oral agonist of MGL-3196, developed by the Madrigal company, has been shown to significantly reduce the LDL-C levels in patients, and ameliorate the metabolic syndromes (such as insulin resistance and dyslipidemia) and fatty liver diseases (including lipid toxicity and inflammation).

In the patent WO 2019240938 A1, a compound of (R)-2-(3,5-dichloro-4-((7-(methyl-d3)-1-oxo-2,5,6,7-tetrahydro-1H-cyclopenta[d]pyridazin-4-yl)oxy)phenyl)-3,5-dioxo-2,3,4,5-tetrahydro-1,2,4-triazine-6-carbonitrile, a thyroxine beta receptor agonist and intended for the treatment of primary hypercholesterolemia, is described and a preparation method therefor is also disclosed. However, there is an urgent problem to be solved in order to develop compounds more suitable for druggable pharmaceutical crystal forms, in particular crystal forms that result in improved stability, hygroscopicity, preservation and/or efficacy, thus achieving good results in the pharmaceutical industry.

### Summary of the Invention

The present invention provides a polymorph of a compound of formula I, which has excellent properties, such as a high purity, good solubility, stable physical and chemical properties, high temperature resistance, high-humidity and strong-light stability, and low hygroscopicity.

A polymorph of a compound of formula I: 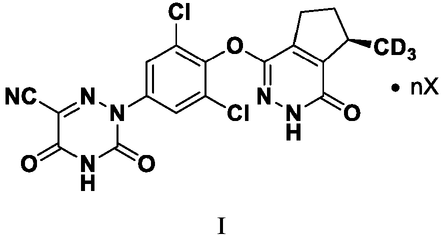 wherein n is 0 to 2, X is H₂O, 4-methyl-2-pentanone, toluene, DMF, acetone, ethyl acetate, DMSO, isopropyl acetate, dioxane, acetonitrile, tetrahydrofuran or ethylene glycol monomethyl ether.

In some embodiments, n is 0 to 2. In some embodiments, n is 0 to 1.

In some embodiments, X is H₂O.

In some embodiments, n is 2, X is H₂O, and the polymorph is of Form A having an X-ray powder diffraction pattern with characteristic diffraction peaks at the following 2θ positions using Cu-Kα radiation: 5.68° ± 0.2°, 11.44° ± 0.2°, 17.19° ± 0.2°, 24.94° ± 0.2°, 26.40° ± 0.2°, 29.47° ± 0.2°.

Further, the Form A, the X-ray powder diffraction pattern of which also has characteristic diffraction peaks at the following 2θ positions: 12.68° ± 0.2°, 19.44° ± 0.2°, 19.79° ± 0.2°, 23.27° ± 0.2°.

Further, the Form A, the X-ray powder diffraction pattern of which has characteristic diffraction peaks at the following 2θ positions:

| Number | Pos. [°2θ] |
|---|---|
| 1 | 5.68 |
| 2 | 11.44 |
| 3 | 12.68 |
| 4 | 14.56 |
| 5 | 16.02 |
| 6 | 17.19 |
| 7 | 19.44 |
| 8 | 19.79 |
| 9 | 22.79 |
| 10 | 23.27 |
| 11 | 24.94 |
| 12 | 25.55 |
| 13 | 25.82 |
| 14 | 26.40 |
| 15 | 29.47 |
| 16 | 35.89 |
| 17 | 36.10 |
| 18 | 41.41 |

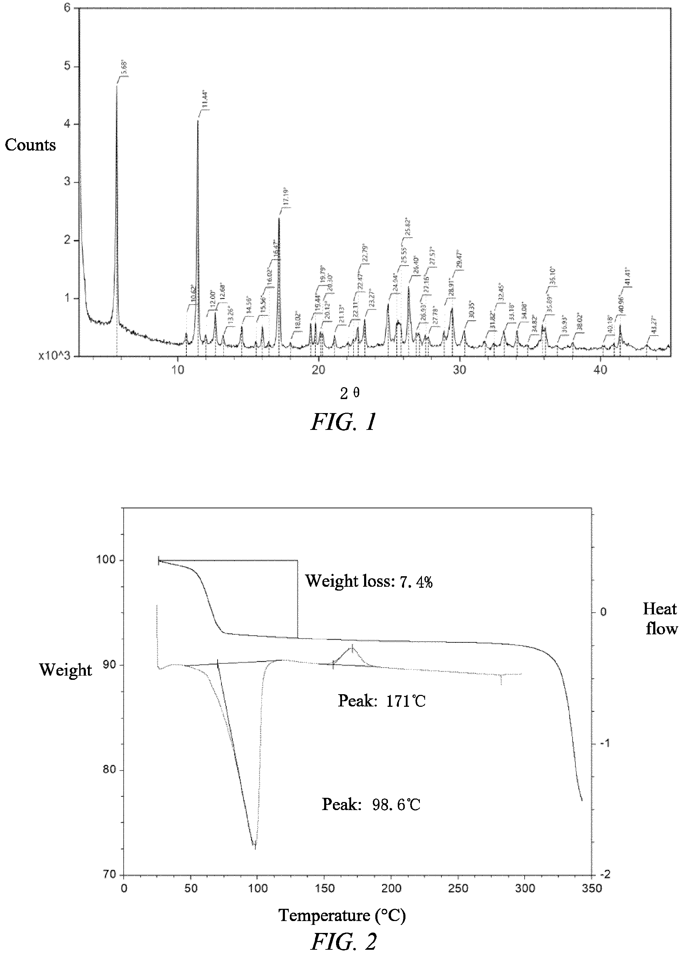

Further, the Form A, the X-ray powder diffraction pattern of which is substantially as shown in Figure 1.

Further, the Form A has a DSC and/or TGA diagram of Figure 2.

In some embodiments, n is 0.4, X is 4-methyl-2-pentanone, and the polymorph is of Form B having an X-ray powder diffraction pattern with characteristic diffraction peaks at the following 2θ positions using Cu-Kα radiation: 8.03° ± 0.2°, 11.05° ± 0.2°, 11.82 ± 0.2°, 16.14 ± 0.2°, 16.55° ± 0.2°, 22.01° ± 0.2°, 22.34° ± 0.2°, 23.43 ± 0.2°, 24.34 ± 0.2°, 25.18° ± 0.2°, 29.30° ± 0.2°.

Further, the Form B, the X-ray powder diffraction pattern of which has characteristic diffraction peaks at the following 2θ positions:

| Number | Pos.[°2θ] |
|---|---|
| 1 | 8.03 |
| 2 | 11.05 |
| 3 | 11.82 |
| 4 | 14.12 |
| 5 | 14.97 |
| 6 | 16.14 |
| 7 | 16.55 |
| 8 | 22.01 |
| 9 | 22.34 |
| 10 | 23.43 |
| 11 | 24.34 |
| 12 | 25.18 |
| 13 | 29.30 |

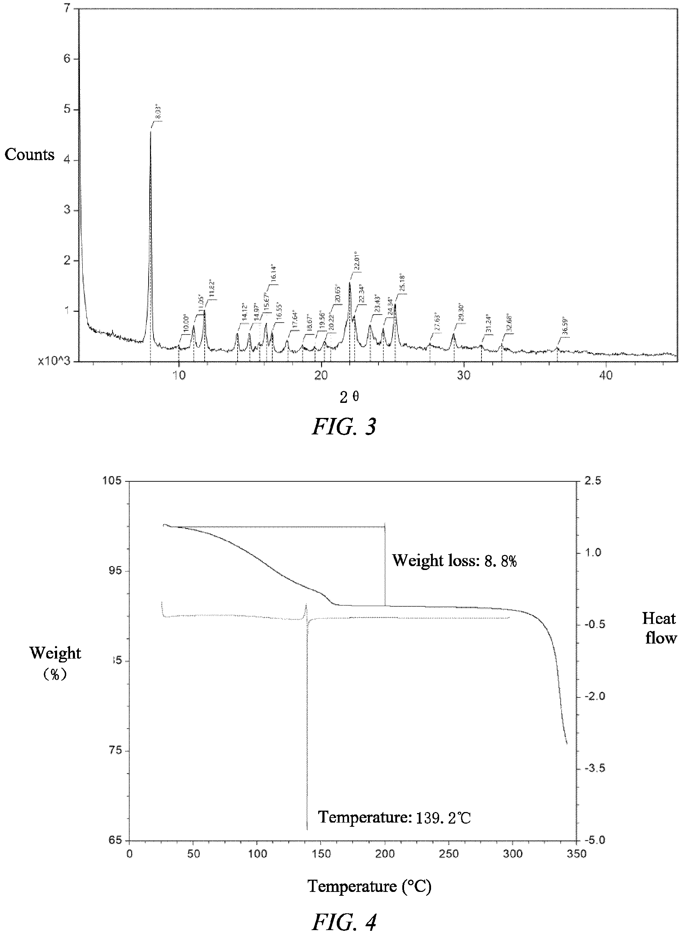

Further, the Form B, X-ray powder diffraction pattern of which is substantially as shown in Figure 3.

Further, the Form B has a DSC and/or TGA diagram of Figure 4.

In some embodiments, n is 0 and the polymorph is of Form C having an X-ray powder diffraction pattern with characteristic diffraction peaks at the following 2θ positions using Cu-Kα radiation: 11.03° ± 0.2°, 15.79° ± 0.2°, 16.97° ± 0.2°, 23.27° ± 0.2°, 23.76° ± 0.2°, 27.61° ± 0.2°.

Further, the Form C, the X-ray powder diffraction pattern of which also has characteristic diffraction peaks at the following 2θ positions: 14.12° ± 0.2°, 19.73° ± 0.2°, 22.51° ± 0.2°, 25.37° ± 0.2°, 28.21° ± 0.2°, 33.73° ± 0.2°.

Further, the Form C, the X-ray powder diffraction pattern of which has characteristic diffraction peaks at the following 2θ positions:

| Number | Pos.[°2θ] |
|---|---|
| 1 | 11.03 |
| 2 | 11.71 |
| 3 | 14.12 |
| 4 | 15.79 |
| 5 | 16.97 |
| 6 | 19.73 |
| 7 | 22.51 |
| 8 | 22.79 |
| 9 | 23.27 |
| 10 | 23.76 |
| 11 | 24.55 |
| 12 | 25.37 |
| 13 | 27.61 |
| 14 | 28.21 |
| 15 | 28.52 |
| 16 | 28.93 |
| 17 | 33.73 |

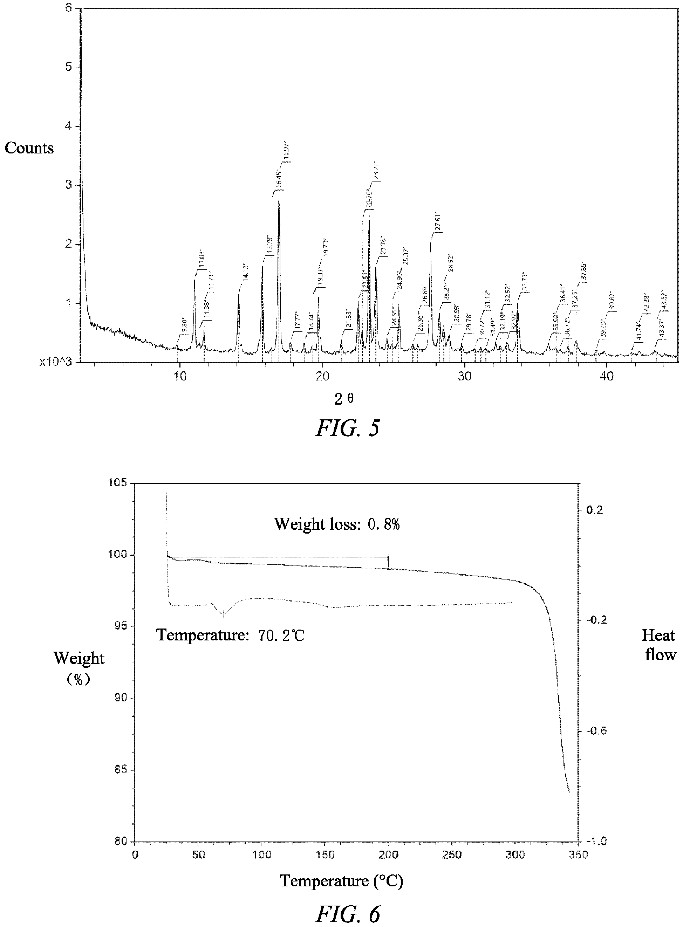

Further, the Form C, the X-ray powder diffraction pattern of which is substantially as shown in Figure 5.

Further, the Form C has a DSC and/or TGA diagram of Figure 6.

In some embodiments, n is 0.4, X is toluene, and the polymorph is of Form D having an X-ray powder diffraction pattern with characteristic diffraction peaks at the following 2θ positions using Cu-Kα radiation: 5.93 ± 0.2°, 11.90° ± 0.2°, 13.38° ± 0.2°, 15.50° ± 0.2°, 16.84° ± 0.2°, 17.91° ± 0.2°, 18.51° ± 0.2°, 20.24° ± 0.2°, 20.86° ± 0.2°, 23.95° ± 0.2°, 26.25° ± 0.2°, 29.94° ± 0.2°, 32.48° ± 0.2°, 36.29° ± 0.2°.

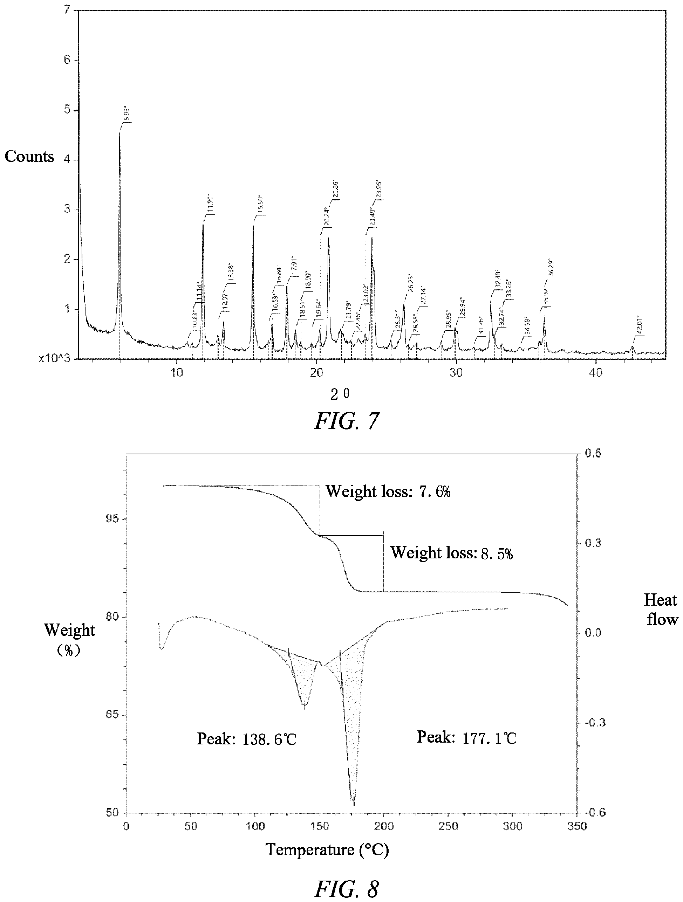

Further, the Form D, the X-ray powder diffraction pattern of which is substantially as shown in Figure 7.

Further, the Form D has a DSC and/or TGA diagram of Figure 8.

In some embodiments, n is 0.8, X is DMF, and the polymorph is of Form E having an X-ray powder diffraction pattern with characteristic diffraction peaks at the following 2θ positions using Cu-Kα radiation: 4.75° ± 0.2°, 5.76° ± 0.2°, 8.23° ± 0.2°, 9.47° ± 0.2°, 10.83° ± 0.2°, 14.21° ± 0.2°, 16.43° ± 0.2°, 18.43° ± 0.2°, 18.70° ± 0.2°, 19.02° ± 0.2°, 22.84° ± 0.2°, 26.42° ± 0.2°.

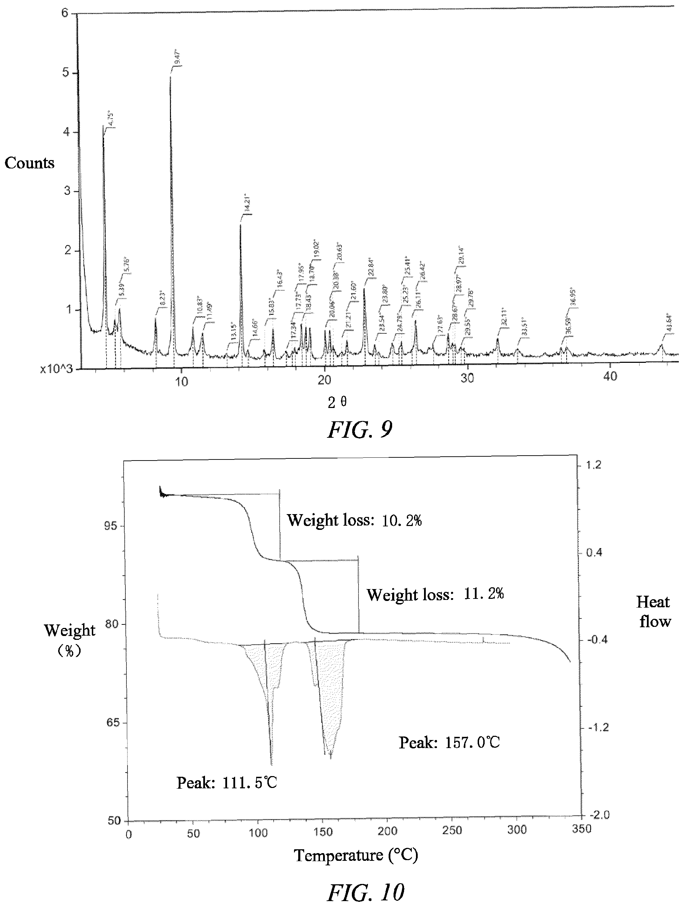

Further, the Form E, the X-ray powder diffraction pattern of which is substantially as shown in Figure 9.

Further, the Form E has a DSC and/or TGA diagram of Figure 10.

In some embodiments, n is 1, X is acetone, and the polymorph is of Form F having an X-ray powder diffraction pattern with characteristic diffraction peaks at the following 2θ positions using Cu-Kα radiation: 6.67° ± 0.2°, 7.65° ± 0.2°, 8.34° ± 0.2°, 10.17° ± 0.2°, 12.37 ± 0.2°, 13.42° ± 0.2°, 15.48° ± 0.2°, 18.49 ± 0.2°, 19.05° ± 0.2°, 20.47° ± 0.2°, 21.64° ± 0.2°, 22.86° ± 0.2°, 23.17° ± 0.2°, 23.41° ± 0.2°, 24.88° ± 0.2°, 28.15° ± 0.2°, 28.75° ± 0.2°, 32.85° ± 0.2°.

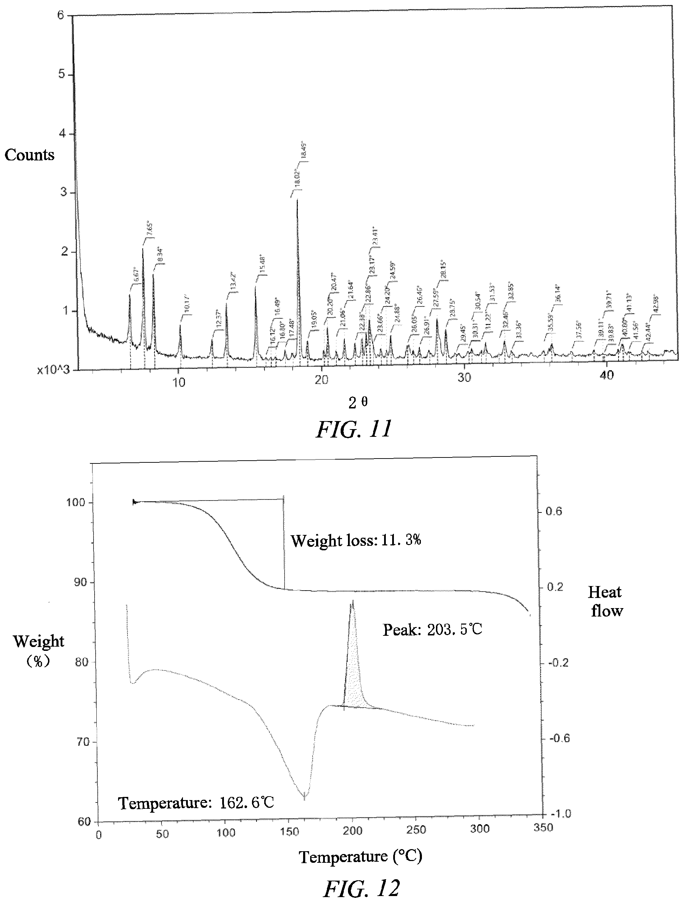

Further, the Form F, the X-ray powder diffraction pattern of which is substantially as shown in Figure 11.

Further, the Form F has a DSC and/or TGA diagram of Figure 12.

In some embodiments, n is 1, X is ethyl acetate, and the polymorph is of Form G having an X-ray powder diffraction pattern with characteristic diffraction peaks at the following 2θ positions using Cu-Kα radiation: 8.69° ± 0.2°, 9.14° ± 0.2°, 11.03° ± 0.2°, 11.88° ± 0.2°, 12.62° ± 0.2°, 13.53° ± 0.2°, 16.14° ± 0.2°, 16.66° ± 0.2°, 16.94° ± 0.2°, 17.40° ± 0.2°, 18.70° ± 0.2°, 18.82° ± 0.2°, 19.19° ± 0.2°, 20.73° ± 0.2°, 22.82° ± 0.2°, 23.76° ± 0.2°, 24.15° ± 0.2°, 24.36° ± 0.2°, 24.79° ± 0.2°, 25.60° ± 0.2°, 26.28° ± 0.2°, 27.16° ± 0.2°, 29.41° ± 0.2°.

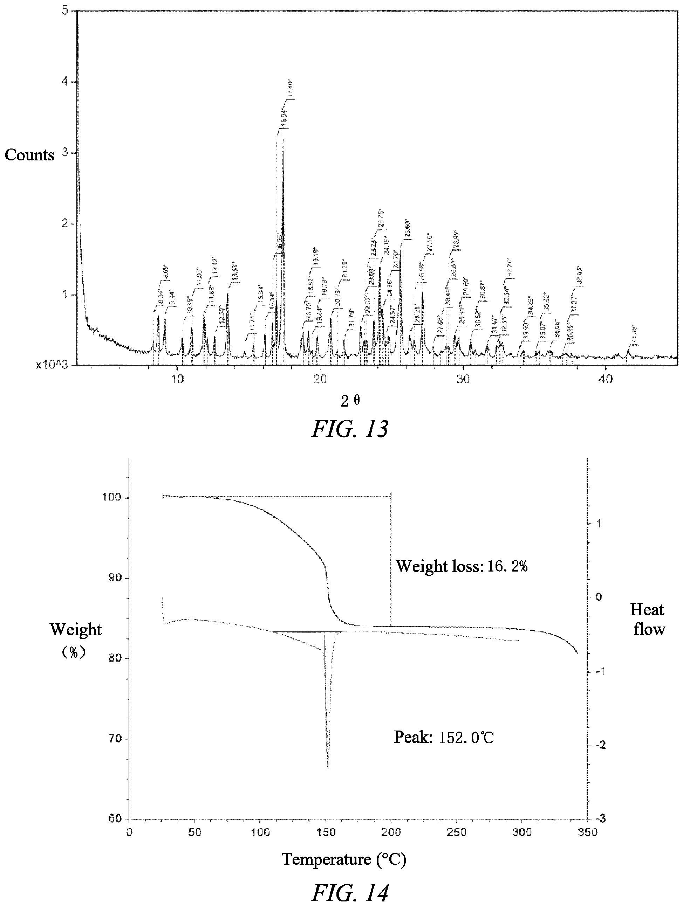

Further, the Form G, X-ray powder diffraction pattern of which is substantially as shown in Figure 13.

Further, the Form G has a DSC and/or TGA diagram of Figure 14.

In some embodiments, n is 1, X is DMSO, and the polymorph is of Form H having an X-ray powder diffraction pattern with characteristic diffraction peaks at the following 2θ positions using Cu-Kα radiation: 5.53° ± 0.2°, 8.54° ± 0.2°, 11.05° ± 0.2°, 16.59° ± 0.2°, 18.16° ± 0.2°, 21.04° ± 0.2°, 21.91° ± 0.2°, 22.16° ± 0.2°, 22.75° ± 0.2°, 23.45° ± 0.2°, 25.70° ± 0.2°, 26.65° ± 0.2°, 27.57° ± 0.2°.

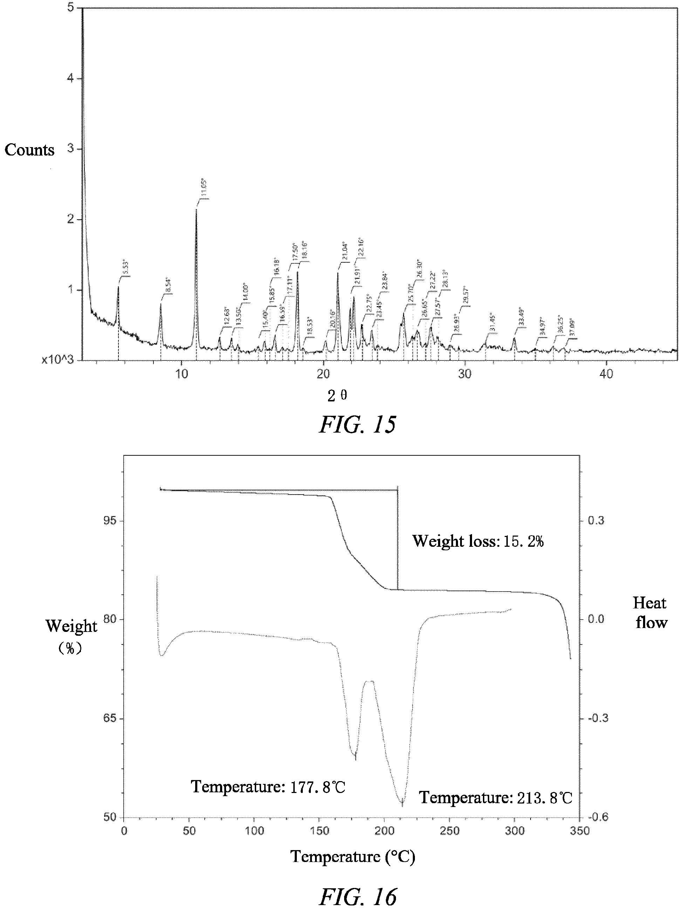

Further, the Form H, the X-ray powder diffraction pattern of which is substantially as shown in Figure 15.

Further, the Form H has a DSC and/or TGA diagram of Figure 16.

In some embodiments, n is 0.9, X is isopropyl acetate, and the polymorph is of Form I having an X-ray powder diffraction pattern with characteristic diffraction peaks at the following 2θ positions using Cu-Kα radiation: 8.36° ± 0.2°, 10.17° ± 0.2°, 11.36° ± 0.2°, 12.72° ± 0.2°, 13.20° ± 0.2°, 16.55° ± 0.2°, 16.78° ± 0.2°, 17.13° ± 0.2°, 18.92° ± 0.2°, 19.85° ± 0.2°, 23.50° ± 0.2°, 23.97° ± 0.2°, 24.50° ± 0.2°, 25.60° ± 0.2°, 26.79° ± 0.2°, 30.00° ± 0.2°.

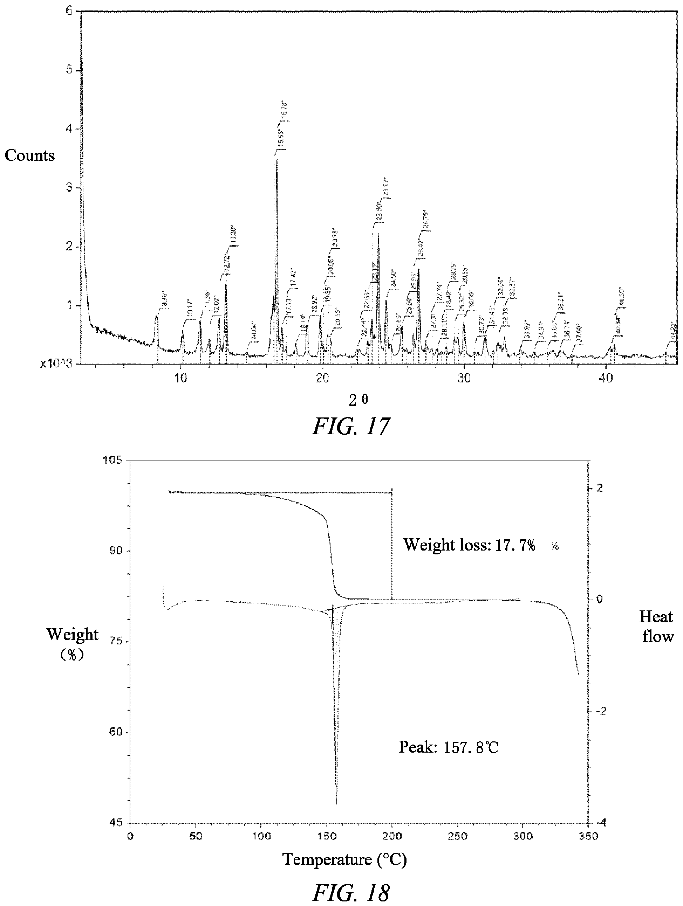

Further, the Form I, the X-ray powder diffraction pattern of which is substantially as shown in Figure 17.

Further, the Form I has a DSC and/or TGA diagram of Figure 18.

In some embodiments, X is acetonitrile and the polymorph is of Form J having an X-ray powder diffraction pattern with characteristic diffraction peaks at the following 2θ positions using Cu-Kα radiation: 10.07° ± 0.2°, 11.07° ± 0.2°, 11.53° ± 0.2°, 13.34° ± 0.2°, 14.08° ± 0.2°, 14.23° ± 0.2°, 14.51° ± 0.2°, 14.99° ± 0.2°, 15.17° ± 0.2°, 15.56° ± 0.2°, 15.83° ± 0.2°, 16.02° ± 0.2°, 16.70° ± 0.2°, 16.99° ± 0.2°, 17.75° ± 0.2°, 17.97° ± 0.2°, 18.35° ± 0.2°, 19.00° ± 0.2°, 20.10° ± 0.2°, 21.97° ± 0.2°, 22.47° ± 0.2°, 23.27° ± 0.2°, 24.11° ± 0.2°, 24.75° ± 0.2°, 25.60° ± 0.2°, 26.32° ± 0.2°, 27..29° ± 0.2°, 27.74° ± 0.2°, 28.66° ± 0.2°, 29.45° ± 0.2°.

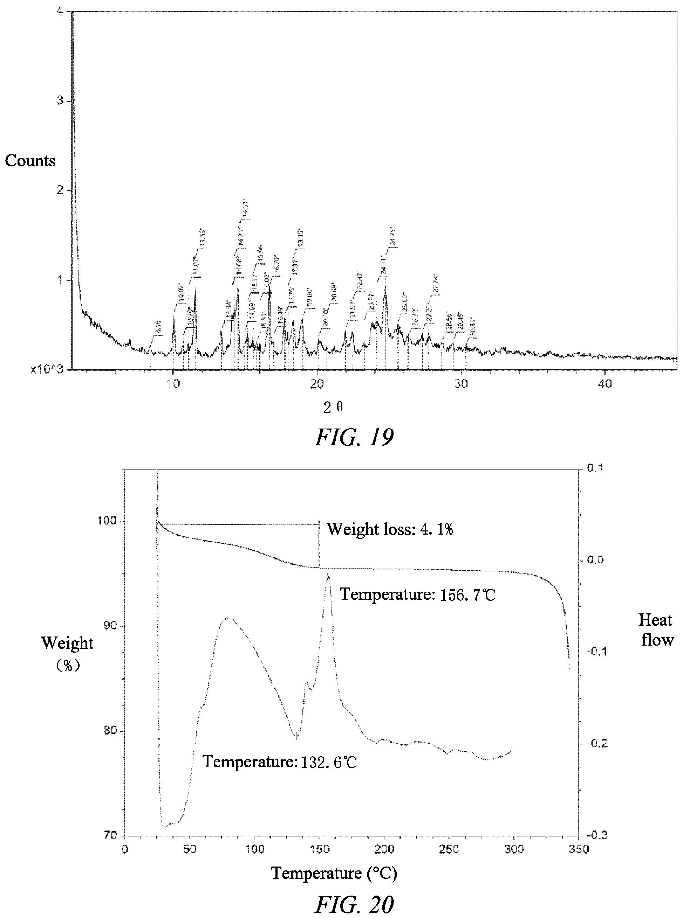

Further, the Form J, the X-ray powder diffraction pattern of which is substantially as shown in Figure 19.

Further, the Form J has a DSC and/or TGA diagram of Figure 20.

In some embodiments, n is 0.9, X is dioxane, and the polymorph is of Form K having an X-ray powder diffraction pattern with characteristic diffraction peaks at the following 2θ positions using Cu-Kα radiation: 7.63° ± 0.2°, 8.34° ± 0.2°, 22.26° ± 0.2°, 23.04° ± 0.2°, 23.27° ± 0.2°.

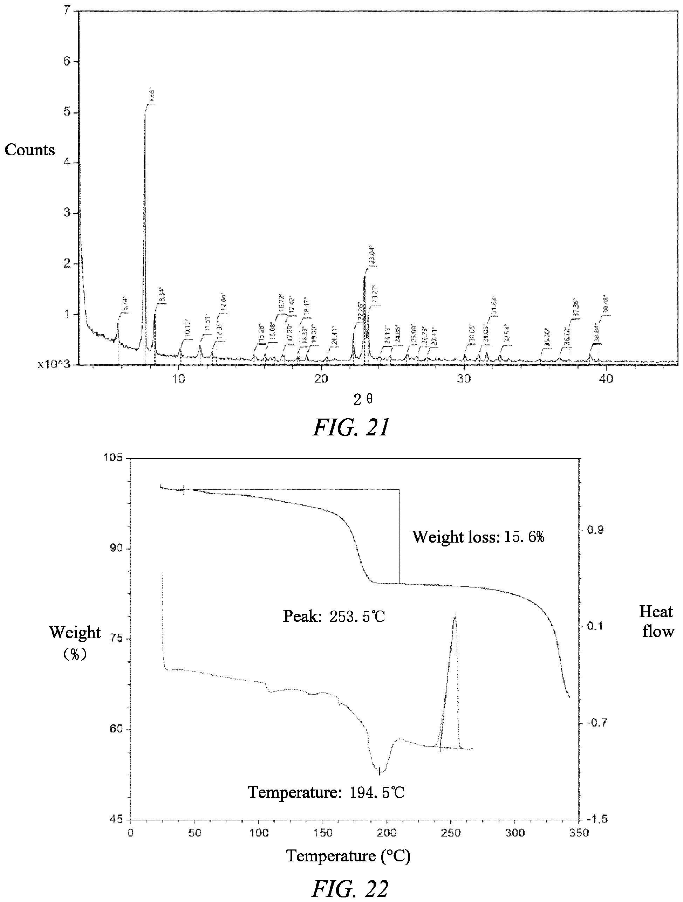

Further, the Form K, the X-ray powder diffraction pattern of which is substantially as shown in Figure 21.

Further, the Form K has a DSC and/or TGA diagram of Figure 22.

In some embodiments, n is 2, X is H₂O, and the polymorph is of Form L having an X-ray powder diffraction pattern with characteristic diffraction peaks at the following 2θ positions using Cu-Kα radiation: 5.72° ± 0.2°, 11.49° ± 0.2°, 12.56° ± 0.2°, 14.33° ± 0.2°, 17.32° ± 0.2°, 25.33° ± 0.2°, 26.25° ± 0.2°.

Further, the Form L, the X-ray powder diffraction pattern of which has characteristic diffraction peaks at the following 2θ positions:

| Number | Pos.[°2θ] |
|---|---|
| 1 | 5.72 |
| 2 | 9.98 |
| 3 | 11.49 |
| 4 | 12.56 |
| 5 | 14.33 |
| 6 | 16.24 |
| 7 | 17.32 |
| 8 | 20.78 |
| 9 | 25.33 |
| 10 | 26.25 |
| 11 | 27.12 |
| 12 | 27.51 |
| 13 | 28.97 |
| 14 | 42.01 |

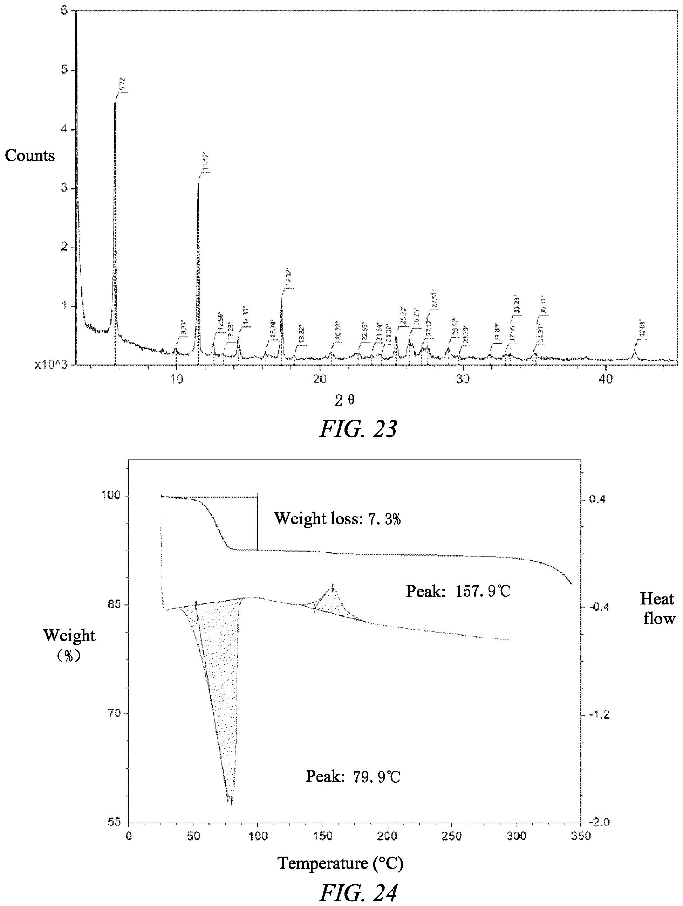

Further, the Form L, the X-ray powder diffraction pattern of which is substantially as shown in Figure 23.

Further, the Form L has a DSC and/or TGA diagram of Figure 24.

In some embodiments, the polymorph is of Form M having an X-ray powder diffraction pattern with characteristic diffraction peaks at the following 2θ positions using Cu-Kα radiation: 4.73° ± 0.2°, 5.24° ± 0.2°, 6.56° ± 0.2°, 7.53° ± 0.2°, 8.31° ± 0.2°, 18.84° ± 0.2°, 21.48° ± 0.2°, 22.88° ± 0.2°.

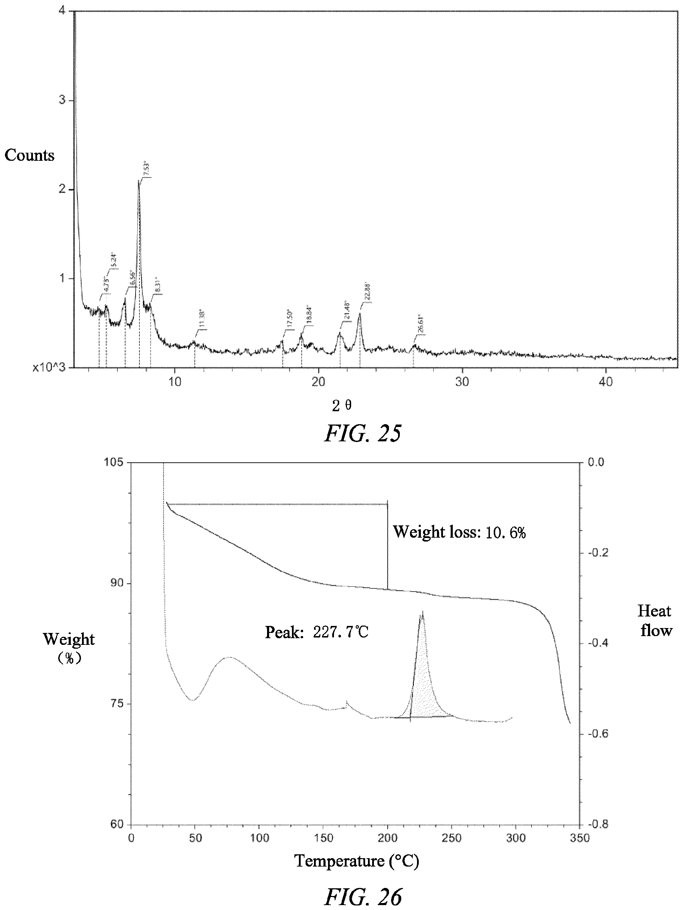

Further, the Form M, the X-ray powder diffraction pattern of which is substantially as shown in Figure 25.

Further, the Form M has a DSC and/or TGA diagram of Figure 26.

In some embodiments, n is 0.6, X is acetonitrile, and the polymorph is of Form N having an X-ray powder diffraction pattern with characteristic diffraction peaks at the following 2θ positions using Cu-Kα radiation: 8.42° ± 0.2°, 9.08° ± 0.2°, 10.85° ± 0.2°, 16.86° ± 0.2°, 17.23° ± 0.2°.

Further, the Form N, the X-ray powder diffraction pattern of which has characteristic diffraction peaks at the following 2θ positions:

| Number | Pos.[°2θ] |
|---|---|
| 1 | 7.65 |
| 2 | 8.42 |
| 3 | 9.08 |
| 4 | 10.85 |
| 5 | 16.86 |
| 6 | 17.23 |

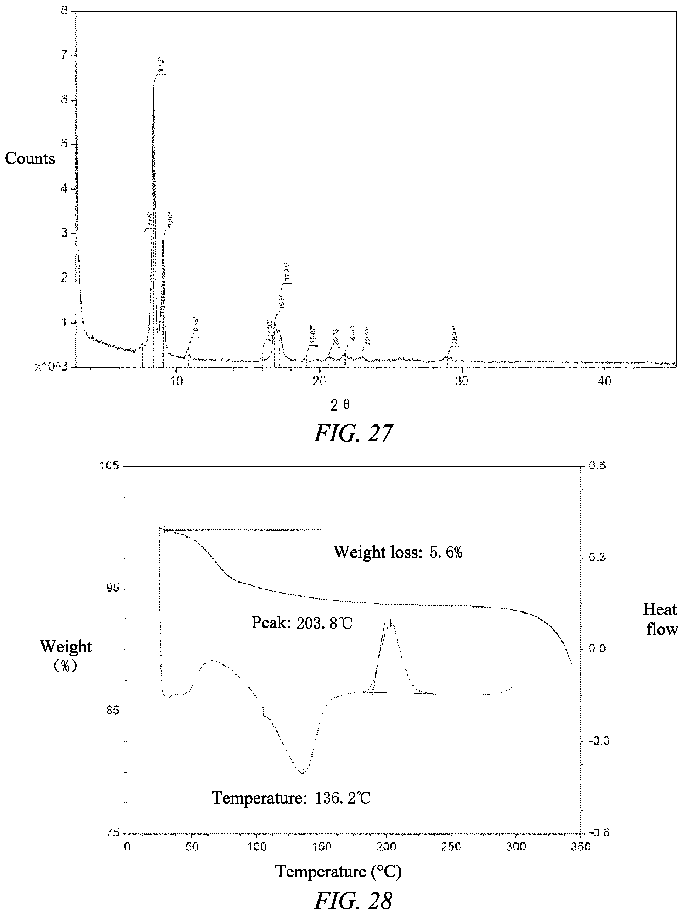

Further, the Form N, the X-ray powder diffraction pattern of which is substantially as shown in Figure 27.

Further, the Form N has a DSC and/or TGA diagram of Figure 28.

In some embodiments, n is 0.8, X is DMF, and the polymorph is of Form O having an X-ray powder diffraction pattern with characteristic diffraction peaks at the following 2θ positions using Cu-Kα radiation: 5.49° ± 0.2°, 8.64° ± 0.2°, 10.89° ± 0.2°, 11.45° ± 0.2°, 16.37° ± 0.2°, 18.55° ± 0.2°, 21.44° ± 0.2°, 22.14° ± 0.2°, 22.73° ± 0.2°, 22.98° ± 0.2°, 23.25° ± 0.2°, 26.17° ± 0.2°.

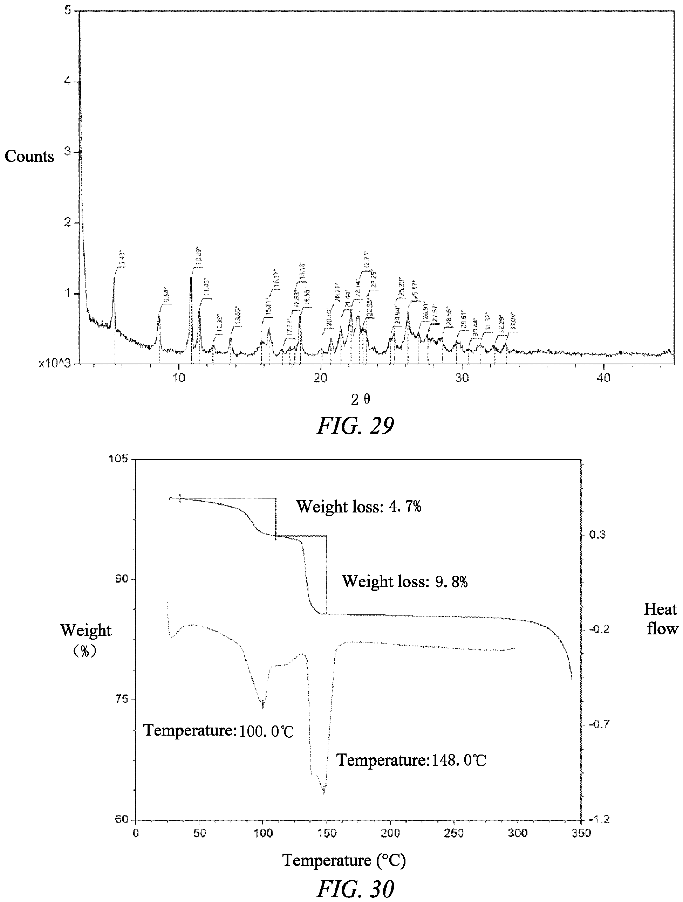

Further, the Form O, the X-ray powder diffraction pattern of which is substantially as shown in Figure 29.

Further, the Form O has a DSC and/or TGA diagram of Figure 30.

In some embodiments, n is 2, X is H₂O, and the polymorph is of Form P having an X-ray powder diffraction pattern with characteristic diffraction peaks at the following 2θ positions using Cu-Kα radiation: 5.76° ± 0.2°, 11.55° ± 0.2°, 17.38° ± 0.2°, 23.12° ± 0.2°, 24.50° ± 0.2°, 26.58° ± 0.2°.

Further, the Form P, the X-ray powder diffraction pattern of which also has characteristic diffraction peaks at the following 2θ positions: 14.60° ± 0.2°, 17.13° ± 0.2°, 25.90° ± 0.2°, 29.48° ± 0.2°.

Further, the Form P, the X-ray powder diffraction pattern of which has characteristic diffraction peaks at the following 2θ positions:

| Number | Pos.[°2θ] |
|---|---|
| 1 | 5.76 |
| 2 | 10.58 |
| 3 | 10.99 |
| 4 | 11.55 |
| 5 | 14.60 |
| 6 | 17.13 |
| 7 | 17.38 |
| 8 | 17.79 |
| 9 | 19.58 |
| 10 | 21.25 |
| 11 | 21.85 |
| 12 | 22.11 |
| 13 | 23.12 |
| 14 | 24.50 |
| 15 | 25.72 |
| 16 | 25.91 |
| 17 | 26.58 |
| 18 | 27.20 |
| 19 | 29.51 |
| 20 | 33.20 |
| 21 | 33.46 |

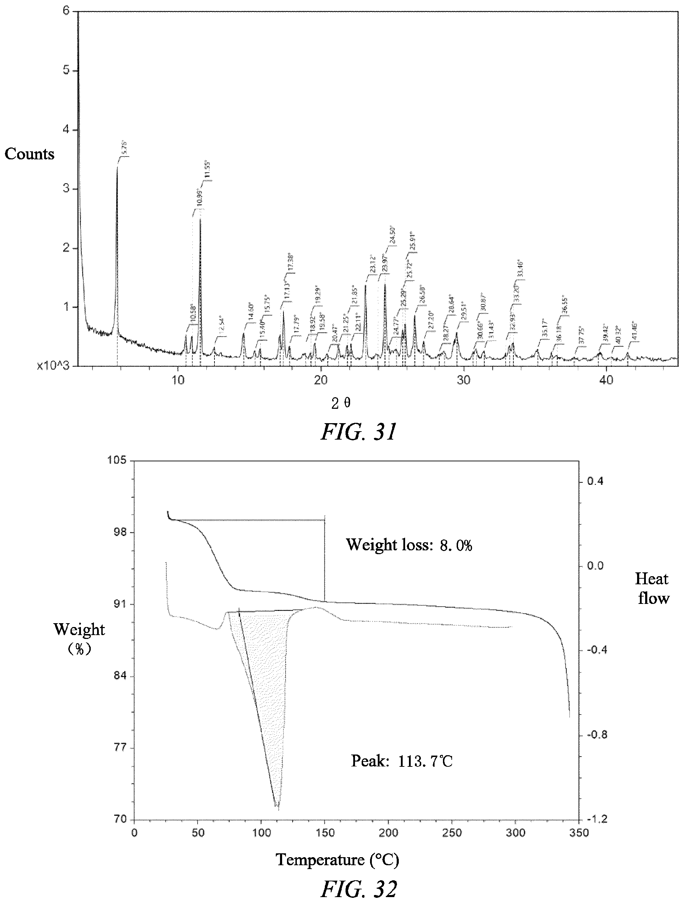

Further, the Form P, the X-ray powder diffraction pattern of which is substantially as shown in Figure 31.

Further, the Form P has a DSC and/or TGA diagram of Figure 32.

In some embodiments, X is water and tetrahydrofuran, and the polymorph is of Form Q having an X-ray powder diffraction pattern with characteristic diffraction peaks at the following 2θ positions using Cu-Kα radiation: 7.55° ± 0.2°, 8.25° ± 0.2°, 10.09° ± 0.2°, 12.29° ± 0.2°, 15.42° ± 0.2°, 18.47° ± 0.2°, 18.96° ± 0.2°, 22.16° ± 0.2°, 22.90° ± 0.2°, 23.17° ± 0.2°, 23.54° ± 0.2°, 24.07° ± 0.2°, 24.79° ± 0.2°, 25.97° ± 0.2°.

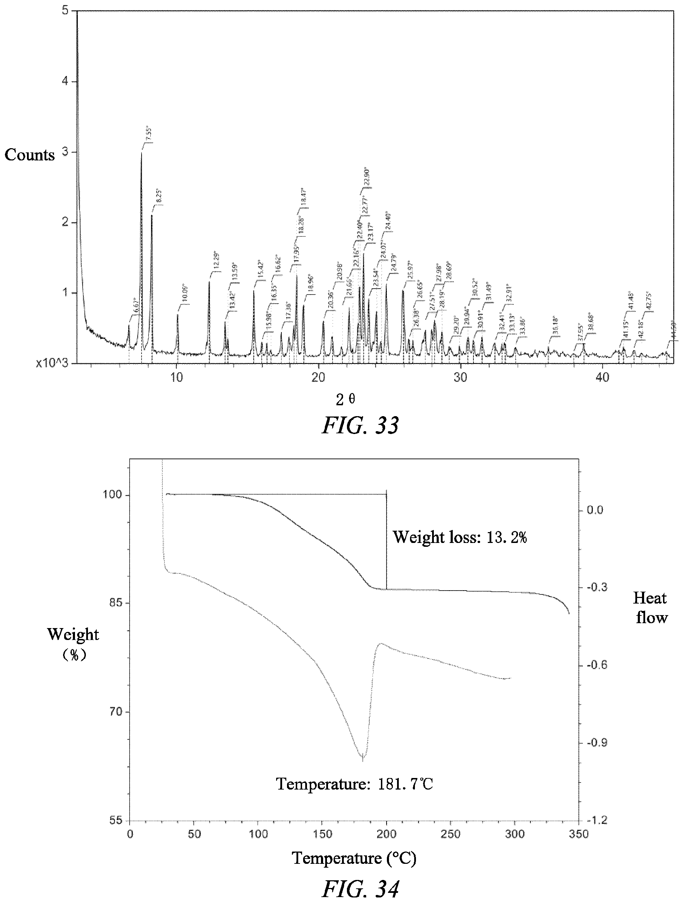

Further, the Form Q, the X-ray powder diffraction pattern of which is substantially as shown in Figure 33.

Further, the Form Q has a DSC and/or TGA diagram of Figure 34.

In some embodiments, n is 0.9, X is dioxane, and the polymorph is of Form R having an X-ray powder diffraction pattern with characteristic diffraction peaks at the following 2θ positions using Cu-Kα radiation: 7.53° ± 0.2°, 8.25° ± 0.2°, 10.07° ± 0.2°, 13.32° ± 0.2°, 15.32° ± 0.2°, 18.33° ± 0.2°, 22.14° ± 0.2°, 22.82° ± 0.2°, 23.14° ± 0.2°.

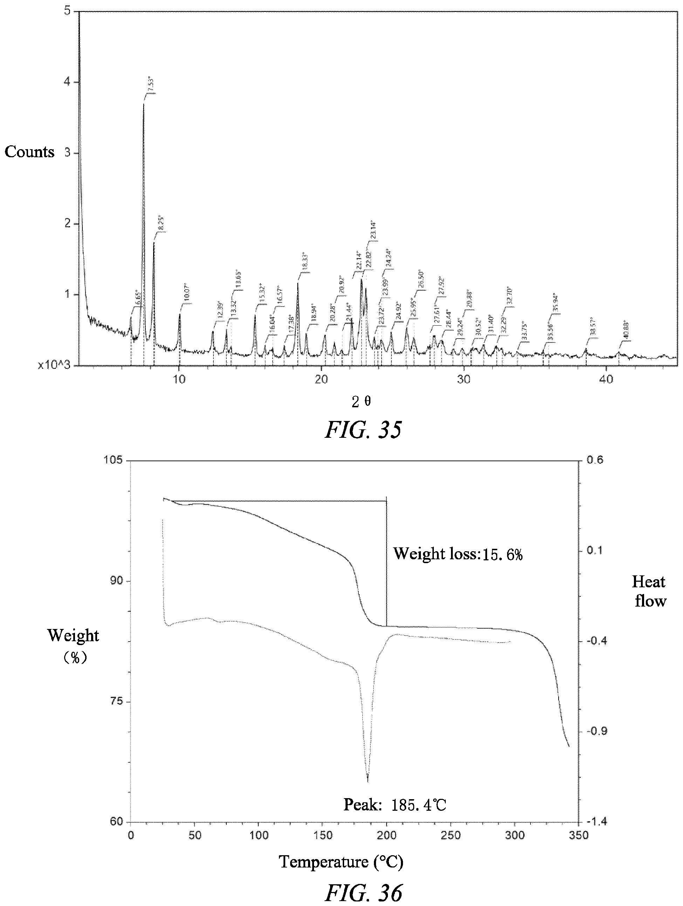

Further, the Form R, the X-ray powder diffraction pattern of which is substantially as shown in Figure 35.

Further, the Form R has a DSC and/or TGA diagram of Figure 36.

In some embodiments, n is 1, X is DMSO, and the polymorph is of Form S having an X-ray powder diffraction pattern with characteristic diffraction peaks at the following 2θ positions using Cu-Kα radiation: 7.74° ± 0.2°, 8.46° ± 0.2°, 10.29° ± 0.2°, 12.37° ± 0.2°, 13.55° ± 0.2°, 15.61° ± 0.2°, 18.65° ± 0.2°, 19.09° ± 0.2°, 23.23° ± 0.2°, 23.49° ± 0.2°, 24.79° ± 0.2°.

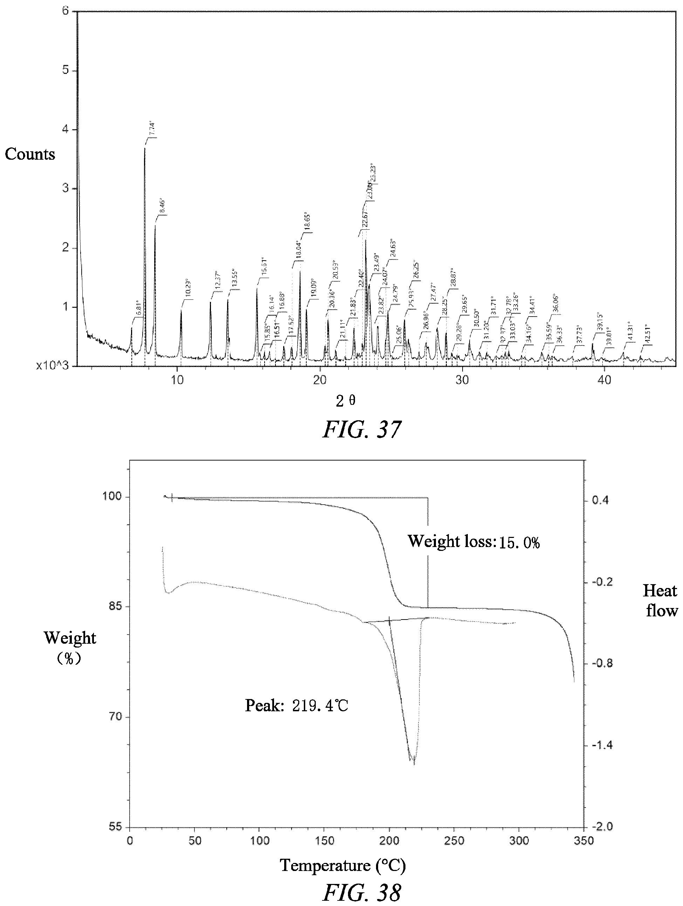

Further, the Form S, the X-ray powder diffraction pattern of which is substantially as shown in Figure 37.

Further, the Form S has a DSC and/or TGA diagram of Figure 38.

In some embodiments, n is 1, X is 4-methyl-2-pentanone, and the polymorph is of Form T having an X-ray powder diffraction pattern with characteristic diffraction peaks at the following 2θ positions using Cu-Kα radiation: 6.81° ± 0.2°, 6.98° ± 0.2°, 7.78° ± 0.2°, 9.76° ± 0.2°, 11.82° ± 0.2°, 13.65° ± 0.2°, 15.36° ± 0.2°, 17.42° ± 0.2°, 18.26° ± 0.2°, 18.51° ± 0.2°, 21.78° ± 0.2°, 22.22° ± 0.2°, 23.82° ± 0.2°, 25.04° ± 0.2°, 28.52° ± 0.2°.

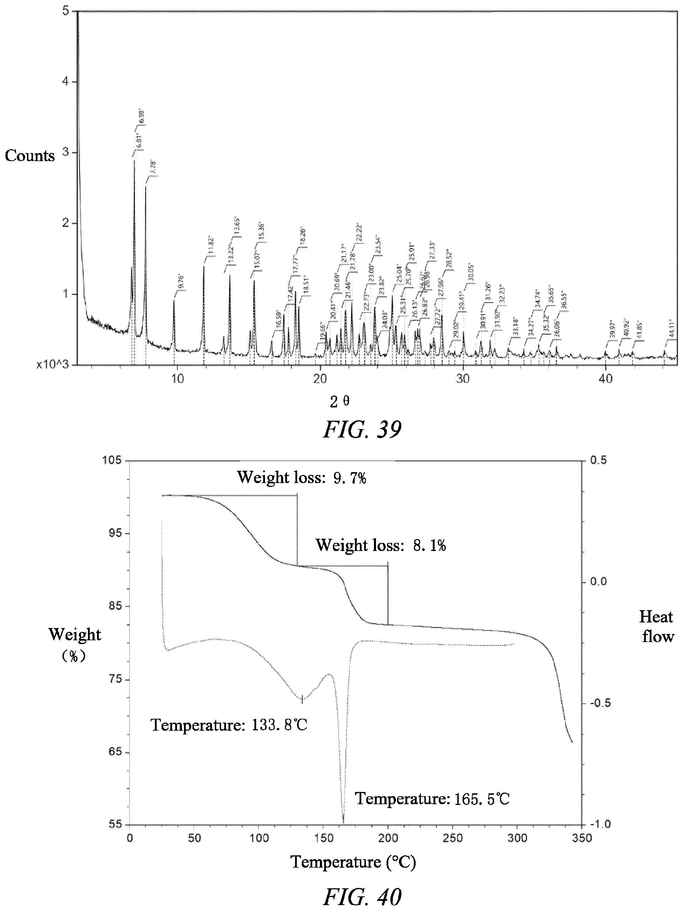

Further, the Form T, the X-ray powder diffraction pattern of which is substantially as shown in Figure 39.

Further, the Form T has a DSC and/or TGA diagram of Figure 40.

In some embodiments, n is 1, X is acetonitrile, and the polymorph is of Form U having an X-ray powder diffraction pattern with characteristic diffraction peaks at the following 2θ positions using Cu-Kα radiation: 7.72° ± 0.2°, 8.42° ± 0.2°, 18.63° ± 0.2°, 23.25° ± 0.2°, 23.49° ± 0.2°.

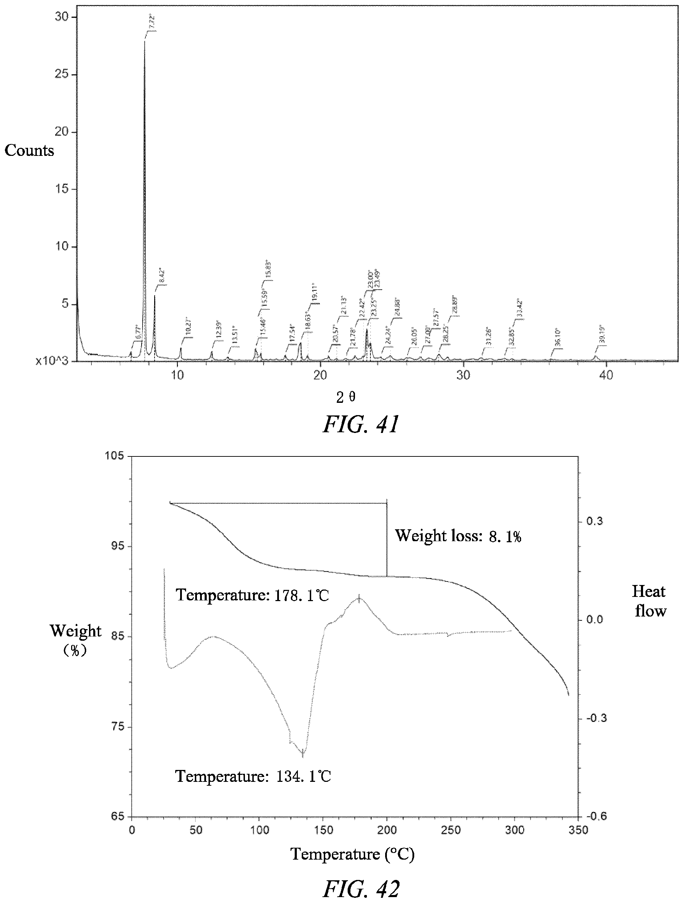

Further, the Form U, the X-ray powder diffraction pattern of which is substantially as shown in Figure 41.

Further, the Form U has a DSC and/or TGA diagram of Figure 42.

In some embodiments, n is 0 and the polymorph is of Form V having an X-ray powder diffraction pattern with characteristic diffraction peaks at the following 2θ positions using Cu-Kα radiation: 7.41° ± 0.2°, 11.05° ± 0.2°, 15.81° ± 0.2°, 16.97° ± 0.2°, 22.53° ± 0.2°, 23.29° ± 0.2°, 27.63° ± 0.2°.

Further, the Form V, the X-ray powder diffraction pattern of which also has characteristic diffraction peaks at the following 2θ positions: 14.16° ± 0.2°, 15.59° ± 0.2°, 19.73° ± 0.2°, 21.33° ± 0.2°, 23.78° ± 0.2°, 25.39° ± 0.2°, 33.75° ± 0.2°.

Further, the Form V, the X-ray powder diffraction pattern of which has characteristic diffraction peaks at the following 2θ positions:

| Number | Pos.[°2θ] |
|---|---|
| 1 | 5.27 |
| 2 | 6.60 |
| 3 | 7.41 |
| 4 | 11.05 |
| 5 | 14.16 |
| 6 | 15.59 |
| 7 | 15.81 |
| 8 | 16.97 |
| 9 | 19.73 |
| 10 | 21.33 |
| 11 | 22.53 |
| 12 | 22.82 |
| 13 | 23.29 |
| 14 | 23.78 |
| 15 | 25.39 |
| 16 | 27.63 |
| 17 | 28.23 |
| 18 | 33.75 |

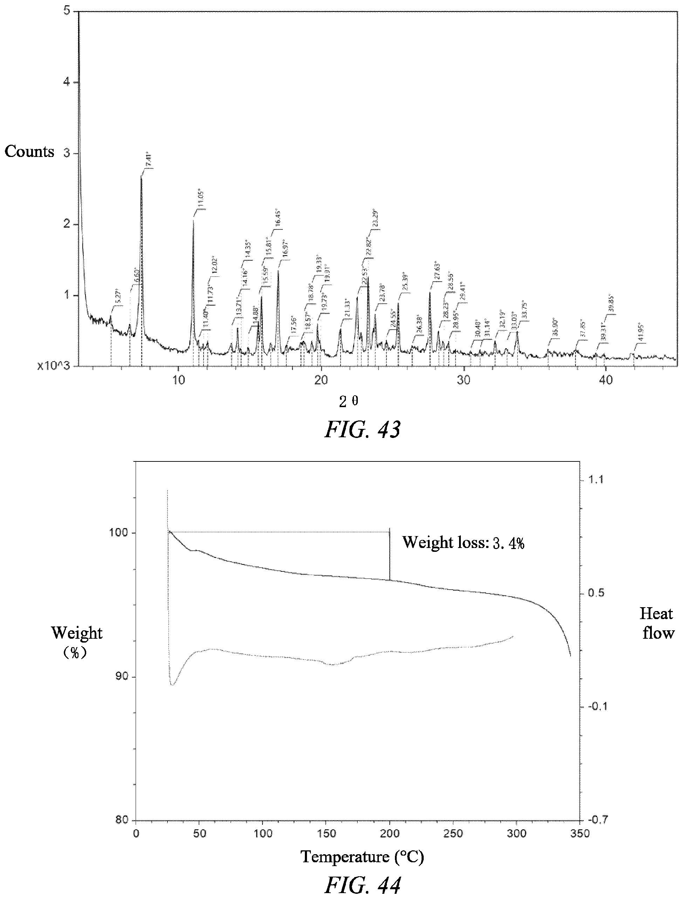

Further, the Form V, the X-ray powder diffraction pattern of which is substantially as shown in Figure 43.

Further, the Form V has a DSC and/or TGA diagram of Figure 44.

In some embodiments, n is 0.7, X is ethylene glycol monomethyl ether, and the polymorph is of Form W having an X-ray powder diffraction pattern with characteristic diffraction peaks at the following 2θ positions using Cu-Kα radiation: 6.69° ± 0.2°, 7.51° ± 0.2°, 8.31° ± 0.2°, 10.17° ± 0.2°, 12.48° ± 0.2°, 13.46° ± 0.2°, 15.52° ± 0.2°, 18.28° ± 0.2°, 18.55° ± 0.2°, 22.20° ± 0.2°, 22.79° ± 0.2°, 23.17° ± 0.2°, 23.89° ± 0.2°, 24.42° ± 0.2°, 25.10° ± 0.2°, 26.65° ± 0.2°, 28.01° ± 0.2°, 28.29° ± 0.2°.

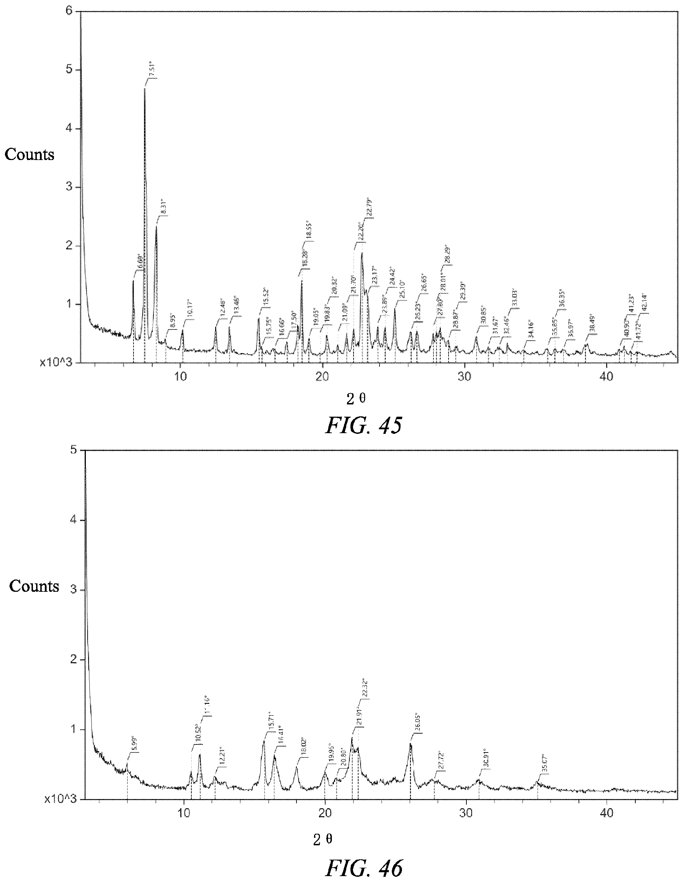

Further, the Form W, the X-ray powder diffraction pattern of which is substantially as shown in Figure 45.

In some embodiments, the polymorph is of Form X having an X-ray powder diffraction pattern with characteristic diffraction peaks at the following 2θ positions using Cu-Kα radiation: 5.99° ± 0.2°, 10.52° ± 0.2°, 11.16° ± 0.2°, 12.21° ± 0.2°, 15.71° ± 0.2°, 16.41° ± 0.2°, 18.02° ± 0.2°, 19.95° ± 0.2°, 21.91° ± 0.2°, 22.32° ± 0.2°, 26.05° ± 0.2°.

Further, the Form X, the X-ray powder diffraction pattern of which is substantially as shown in Figure 46.

In some embodiments, the polymorph is of Form Y having an X-ray powder diffraction pattern with characteristic diffraction peaks at the following 2θ positions using Cu-Kα radiation: 3.21° ± 0.2°, 3.23° ± 0.2°, 7.66° ± 0.2°, 8.36° ± 0.2°, 9.08° ± 0.2°, 10.25° ± 0.2°, 12.43° ± 0.2°, 18.61° ± 0.2°, 19.03° ± 0.2°, 22.98° ± 0.2°, 23.27° ± 0.2°, 23.70° ± 0.2°, 24.92° ± 0.2°, 26.13° ± 0.2°.

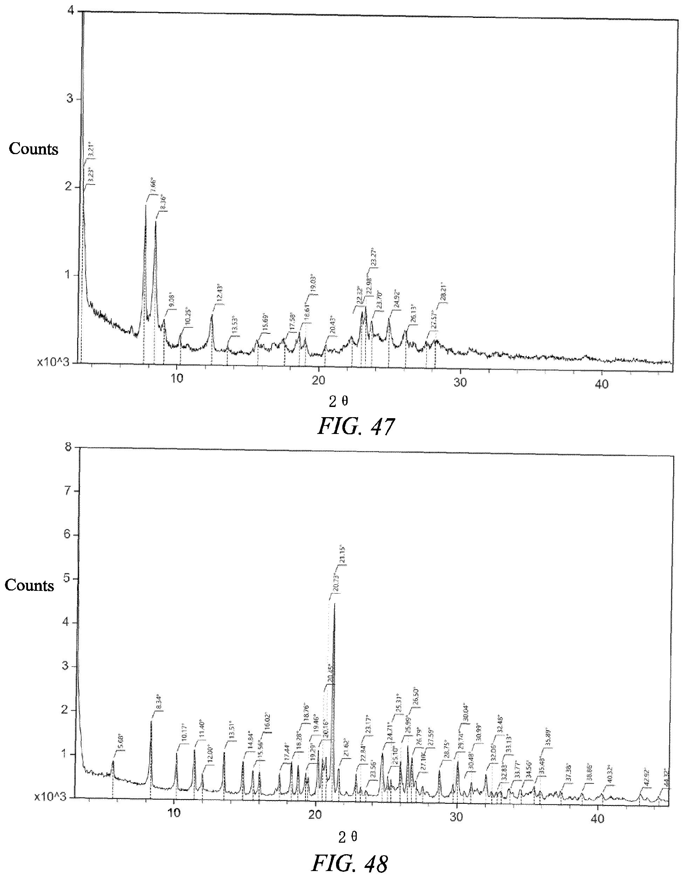

Further, the Form Y, the X-ray powder diffraction pattern of which is substantially as shown in Figure 47.

In some embodiments, n is 0.9, X is DMF, and the polymorph is of Form Z having an X-ray powder diffraction pattern with characteristic diffraction peaks at the following 2θ positions using Cu-Kα radiation: 8.34° ± 0.2°, 10.17° ± 0.2°, 11.40° ± 0.2°, 13.51° ± 0.2°, 14.84° ± 0.2°, 18.28° ± 0.2°, 18.76° ± 0.2°, 20.16° ± 0.2°, 20.45° ± 0.2°, 20.73° ± 0.2°, 21.15° ± 0.2°, 24.71° ± 0.2°, 25.99° ± 0.2°, 26.50° ± 0.2°, 26.79° ± 0.2°, 30.04° ± 0.2°.

Further, the Form Z, the X-ray powder diffraction pattern of which is substantially as shown in Figure 48.

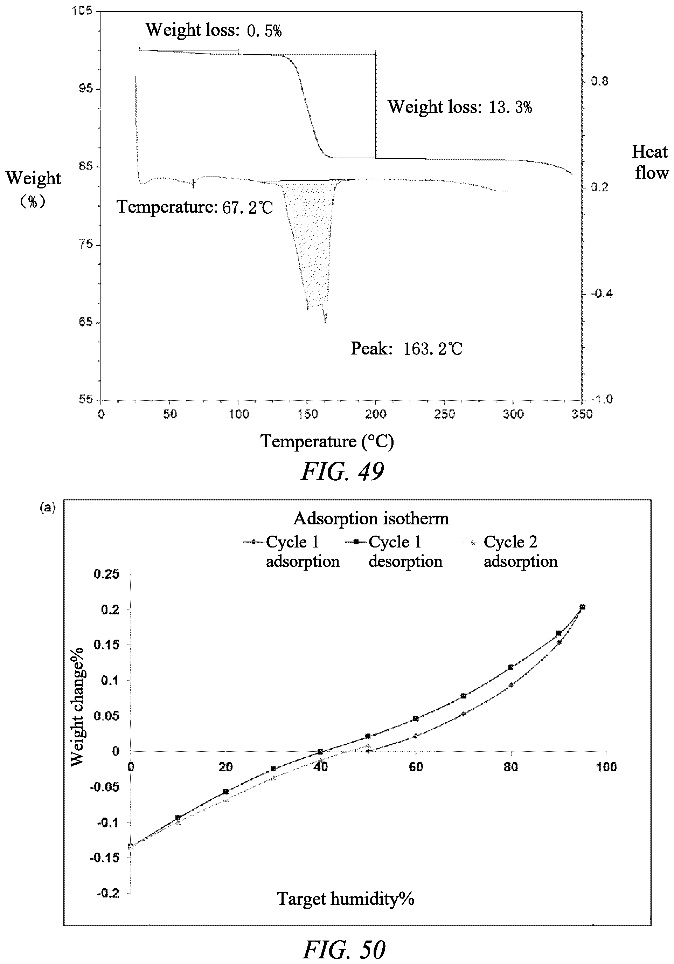

Further, the Form Z has a DSC and/or TGA diagram of Figure 49.

The present invention further provides a pharmaceutical composition comprising the above polymorph of any one of the Form A to Form Z, and a pharmaceutically acceptable carrier and/or excipient. In some embodiments, the pharmaceutical composition comprises the above polymorph of any one of Forms A, C, L, P, and V, and a pharmaceutically acceptable carrier and/or excipient.

The present invention further provides the use of the above polymorph of any one of the Form A to Form Z, or a composition comprising same, in the manufacture of a medicament for the treatment of a disease mediated by the thyroid hormone β receptor. In some embodiments, provided is the use of the above polymorph of any one of the Forms A, C, L, P, and V, or a composition comprising same, in the manufacture of a medicament for the treatment of a disease mediated by the thyroid hormone β receptor.

Further, the disease mediated by the thyroid hormone β receptor is primary hypercholesterolemia.

The polymorph of Form A to Form Z of the present invention is present in an amount from about 5% to about 100% by weight of active pharmaceutical ingredients; in certain embodiments, in an amount from about 10% to about 100% by weight of the active pharmaceutical ingredients; in certain embodiments, in an amount from 15% to about 100% by weight of the active pharmaceutical ingredients; in certain embodiments, in an amount from about 20% to about 100% by weight of the active pharmaceutical ingredients; in certain embodiments, in an amount from about 25% to about 100% by weight of the active pharmaceutical ingredients; in certain embodiments, in an amount from about 30% to about 100% by weight of the active pharmaceutical ingredients; in certain embodiments, in an amount from about 35% to about 100% by weight of the active pharmaceutical ingredients; in certain embodiments, in an amount from about 40% to about 100% by weight of the active pharmaceutical ingredients; in certain embodiments, in an amount from about 45% to about 100% by weight of the active pharmaceutical ingredients; in certain embodiments, in an amount from about 50% to about 100% by weight of the active pharmaceutical ingredients; in certain embodiments, in an amount from about 55% to about 100% by weight of the active pharmaceutical ingredients; in certain embodiments, in an amount from about 60% to about 100% by weight of the active pharmaceutical ingredients; in certain embodiments, in an amount from about 65% to about 100% by weight of the active pharmaceutical ingredients; in certain embodiments, in an amount from about 70% to about 100% by weight of the active pharmaceutical ingredients; in certain embodiments, in an amount from about 75% to about 100% by weight of the active pharmaceutical ingredients; in certain embodiments, in an amount from about 80% to about 100% by weight of the active pharmaceutical ingredients; in certain embodiments, in an amount from about 85% to about 100% by weight of the active pharmaceutical ingredients; in certain embodiments, in an amount from about 90% to about 100% by weight of the active pharmaceutical ingredients; in certain embodiments, in an amount from about 95% to about 100% by weight of the active pharmaceutical ingredients; in certain embodiments, in an amount from about 98% to about 100% by weight of the active pharmaceutical ingredients; in certain embodiments, in an amount from about 99% to about 100% by weight of the active pharmaceutical ingredients; and in some embodiments, substantially all of the active pharmaceutical ingredients are substantially pure crystals.

The present invention further provides a method for preparing the polymorph of any one of Form A to Form Z, including:
(1) suspension method: adding a crude free base of the compound of formula I (which can be prepared according to the methods described in WO 2019240938 A1) to a selected single or binary solvent until a suspension is formed and stirring same under suspension at room temperature to 50°C for a period of time (e.g., 1 h to 3 days, or 2 h to 24 h, or 2 h to 12 h, or 3 h to 5 h), and then centrifuging and separating the suspension, followed by drying, to obtain the product; or
(2) Anti-solvent crystallization method: adding a crude free base of the compound of formula I to an appropriate amount of a good solvent until completely dissolved, taking an amount of the solution and dropwise adding same to a poor solvent or dropwise adding a poor solvent to the solution, and stirring same to precipitate a solid, followed by separation and drying to obtain the product; or
(3) cooling method: adding a crude free base of the compound of formula I to an appropriate amount of a solvent at 50-80°C until the solid is just completely dissolved, and transferring the solution to an environment at room temperature for cooling, followed by leaving to stand or crystallization under stirring, separation and drying to obtain the product; or
(4) vapor diffusion method: dropwise adding an appropriate amount of a good solvent to an amount of a crude free base of the compound of formula I at room temperature to completely dissolve the sample or formulating same as a saturated solution of a good solvent; and separately taking an amount of the solution, and leaving the clear solution to stand in a poor solvent atmosphere at room temperature until a solid precipitates out, followed by separation, to obtain the product; or
(5) thermal crystal transformation method: placing an amount of the sample on a glass plate on a hot stage, heating same to the target temperature at a rate (e.g., 5-20°C/min, or 10-15°C/min), and maintaining the temperature for a period of time (e.g., 0.5-5 min, or 1-3 min, or 1-2 min), followed by naturally cooling down to room temperature to obtain a solid.

The good and poor solvents according to the present invention are relative; and in a pair of solvents, the one with a higher solubility is a good solvent and the one with a lower solubility is a poor solvent. In some embodiments, the good solvent is selected from ethylene glycol methyl ether, ethylene glycol dimethyl ether, dioxane, DMF, DMSO, methanol, ethanol, n-propanol, butyl formate, 4-methyl-2-pentanone, tetrahydrofuran, isopropanol, ethyl acetate, n-heptane, diethyl ether, water, acetonitrile, toluene, chloroform, acetone, butyl formate, MTBE, and cyclohexane with a higher solubility, and the poor solvent is selected from the above solvents with a lower solubility. In some embodiments, the good solvent is selected from ethylene glycol methyl ether, ethylene glycol dimethyl ether, dioxane, DMF, DMSO, methanol, ethanol, n-propanol, butyl formate, 4-methyl-2-pentanone, tetrahydrofuran or a mixture solvent thereof. In some embodiments, the poor solvent is selected from isopropanol, ethyl acetate, n-heptane, diethyl ether, water, acetonitrile, toluene, chloroform, acetone, butyl formate, MTBE, cyclohexane or a mixture solvent thereof.

The solvents used in the above five preparation methods may be a single solvent or a combination of two or more solvents, if not specified.

In some embodiments, the solvent in the suspension method is chloroform. In some embodiments, the solvent in the suspension method is tetrahydrofuran. In some embodiments, the solvent in the suspension method is a combination of 4-methyl-2-pentanone and cyclohexane. In some embodiments, the solvent in the suspension method is isopropyl acetate. In some embodiments, the solvent in the suspension method is acetonitrile. In some embodiments, the solvent in the suspension method is chloroform and ethanol. In some embodiments, the solvent in the suspension method is toluene. In some embodiments, the solvent in the suspension method is water and DMF.

In some embodiments, the solvent used in the anti-solvent crystallization method is ethylene glycol methyl ether and toluene. In some embodiments, the solvent used in the anti-solvent crystallization method is DMF and water. In some embodiments, the solvent used in the anti-solvent crystallization method is ethylene glycol monomethyl ether and acetone. In some embodiments, the solvent used in the anti-solvent crystallization method is dioxane and diethyl ether. In some embodiments, the solvent used in the anti-solvent crystallization method is ethylene glycol methyl ether and ethyl acetate. In some embodiments, the solvent used in the anti-solvent crystallization method is DMSO and water. In some embodiments, the solvent used in the anti-solvent crystallization method is dioxane and n-heptane. In some embodiments, the solvent used in the anti-solvent crystallization method is dioxane and water. In some embodiments, the solvent used in the anti-solvent crystallization method is ethylene glycol dimethyl ether and chloroform.

In some embodiments, the solvent used in the cooling method is ethanol and DMSO. In some embodiments, the solvent used in the cooling method is acetonitrile and methanol. In some embodiments, the solvent used in the cooling method is ethylene glycol dimethyl ether and acetonitrile. In some embodiments, the solvent used in the cooling method is methanol and DMF. In some embodiments, the solvent used in the cooling method is toluene and methanol.

In some embodiments, the vapor diffusion method involves the vapor diffusion of an ethylene glycol monomethyl ether solution in diethyl ether.

The present invention has the following beneficial effects:
the crystal form of the present invention has excellent physicochemical properties, such as, good flowability and significantly improved viscosity, etc., so that the crystal form of the present invention can significantly reduce the filtration time, shorten the production cycle, and save costs when being used to prepare a formulation; good photostability, thermal stability and wet stability can ensure the reliability of the crystal form during storage and transportation, thus ensuring the safety and efficacy of the formulation after long-term storage, and the crystal form does not require special packaging treatments to prevent exposure to light, temperature and humidity, thereby reducing costs; the crystal form is not degraded by light, high temperature, or high humidity, and patients taking the crystal form do not need to worry about the photosensitive reaction of the formulation due to exposure to sunlight; and the crystal form also has a high solubility, which facilitates the drug dissolution and improves the bioavailability.

The polymorphs of the present invention, particularly Form C, have XRPD results showing good crystalline solids with very good stability. The crystal form has a high melting point, good thermal stability, and no crystal form transformation under high temperature, high humidity, light, and accelerated conditions, and many forms give more stable Form C after crystal transformation, with little hygroscopicity. Such a form is very useful as an active ingredient in pharmaceutical formulations, stable even when stored at temperatures above room temperature, and also stable during the preparation of formulations at high temperatures.

It can be understood that, as is well known in the art of differential scanning calorimetry (DSC), the height of melting peaks of a DSC curve depends on many factors related to sample preparation and geometric shapes of instruments, while the position of the peaks is relatively insensitive to experiment details. Therefore, in some embodiments, the crystallised compounds of the present invention have DSC patterns showing characteristic peak positions, which have essentially the same properties as the DSC patterns provided in the drawings of the present invention, with an error tolerance of measured values within ± 5°C, which is generally required to be within ± 3°C.

It can be understood that the numerical values described and claimed in the present invention are approximate values. Changes in values may be attributed to device calibration, device errors, crystal purity, crystal size, sample size and other factors.

It can be understood that the crystal forms of the present invention are not limited to the characteristic patterns such as XRPD, DSC, TGA, DVS and adsorption isotherm curve graphs which are completely identical to those described in the drawings disclosed by the present invention, and any crystal form having a characteristic pattern which is essentially or essentially the same as those described in the drawings falls within the scope of the present invention.

The "therapeutically effective amount" means an amount that causes a physiological or medical response in a tissue, system or subject and is a desirable amount, including the amount of a compound that is, when administered to a subject to be treated, sufficient to prevent occurrence of one or more symptoms of the disease or condition to be treated or to reduce the symptom(s) to a certain degree.

The "room temperature" refers to 10°C-30°C.

The term "carrier" refers to: a system that does not cause significant irritation to the organism and does not eliminate the biological activity and characteristics of the administered compound, and can change the way the drug enters the human body and the distribution of the drug in the body, control the release rate of the drug and delivery the drug to targeted organs. Non-limiting examples of the carrier include microcapsule, microsphere, nanoparticle, liposome, etc.

The term "excipient" refers to: a substance that is not a therapeutic agent per se, but used as a diluent, adjuvant, adhesive and/or vehicle for addition to a pharmaceutical composition, thereby improving the disposal or storage properties thereof, or allowing to or promoting the formation of a compound or a pharmaceutical composition into a unit dosage form for administration. As is known to those skilled in the art, a pharmaceutically acceptable excipient can provide various functions and can be described as a wetting agent, a buffer, a suspending agent, a lubricant, an emulsifier, a disintegrating agent, an absorbent, a preservative, a surfactant, a colorant, a flavouring agent and a sweetening agent. Examples of pharmaceutically acceptable excipients include, but are not limited to: (1) sugars, such as lactose, glucose and sucrose; (2) starch, such as corn starch and potato starch; (3) cellulose and its derivatives, such as sodium carboxymethyl cellulose, ethyl cellulose, cellulose acetate, hydroxypropyl methylcellulose, hydroxypropyl cellulose, microcrystalline cellulose and croscarmellose (such as croscarmellose sodium); (4) tragacanth powder; (5) malt; (6) gelatine; (7) talc; (8) excipients, such as cocoa butter or suppository wax; (9) oils, such as peanut oil, cottonseed oil, safflower oil, sesame oil, olive oil, corn oil and soybean oil; (10) diols, such as propylene glycol; (11) polyols, such as glycerol, sorbitol, mannitol and polyethylene glycol; (12) esters, such as ethyl oleate and ethyl laurate; (13) agar; (14) buffers, such as magnesium hydroxide and aluminium hydroxide; (15) alginic acid; (16) pyrogen-free water; (17) isotonic saline; (18) Ringer's solution; (19) ethanol; (20) pH buffered solution; (21) polyester, polycarbonate and/or polyanhydride; and (22) other non-toxic compatible substances used in a pharmaceutical formulation.

"Crystal form" or "crystal" or "polymorph" refers to any solid substance that exhibits a three-dimensional order and, in contrast to an amorphous solid substance, results in a characteristic XRPD pattern with well-defined peaks.

"X-ray powder diffraction pattern (XRPD pattern)" refers to an experimentally observed diffraction pattern or parameters, data or values derived therefrom. XRPD patterns are typically characterised by peak positions (abscissa) and/or peak intensities (ordinate).

"2θ" refers to the position of the peak in degrees (°) set in X-ray diffraction experiments, and is usually the abscissa unit in the diffraction pattern. If the reflection is diffracted when the incident beam forms a θ angle with a lattice plane, the experimental setup requires that the reflected beam be recorded at a 2θ angle. It should be understood that the specific 2θ value of a particular form referred to herein is intended to represent the 2θ value (in degrees) measured using the X-ray diffraction experimental conditions described herein.

The terms "substantially the same" or "substantially as shown in Figure XX" for X-ray diffraction peaks mean that representative peak positions and intensity variations are taken into account. For example, those skilled in the art will appreciate that the peak position (2θ) may have some variation, typically up to 0.1-0.2°, and that the instrument used to measure diffraction may also cause some variation. In addition, those skilled in the art will appreciate that relative peak intensities may vary due to instrument-to-instrument differences as well as degree of crystallinity, preferential orientation, prepared sample surfaces, and other factors known to those skilled in the art and should be considered as a qualitative measurement only.

### Brief Description of the Drawings

Figure 1 shows the X-ray powder diffraction pattern of Form A of the compound of formula I.
Figure 2 shows the Differential Scanning Calorimetry and Thermogravimetric Analysis of Form A of the compound of formula I.
Figure 3 shows the X-ray powder diffraction pattern of Form B of the compound of formula I.
Figure 4 shows the Differential Scanning Calorimetry and Thermogravimetric Analysis of Form B of the compound of formula I.
Figure 5 shows the X-ray powder diffraction pattern of Form C of the compound of formula I.
Figure 6 shows the Differential Scanning Calorimetry and Thermogravimetric Analysis of Form C of the compound of formula I.
Figure 7 shows the X-ray powder diffraction pattern of Form D of the compound of formula I.
Figure 8 shows the Differential Scanning Calorimetry and Thermogravimetric Analysis of Form D of the compound of formula I.
Figure 9 shows the X-ray powder diffraction pattern of Form E of the compound of formula I.
Figure 10 shows the Differential Scanning Calorimetry and Thermogravimetric Analysis of Form E of the compound of formula I.
Figure 11 shows the X-ray powder diffraction pattern of Form F of the compound of formula I.
Figure 12 shows the Differential Scanning Calorimetry and Thermogravimetric Analysis of Form F of the compound of formula I.
Figure 13 shows the X-ray powder diffraction pattern of Form G of the compound of formula I.
Figure 14 shows the Differential Scanning Calorimetry and Thermogravimetric Analysis of Form G of the compound of formula I.
Figure 15 shows the X-ray powder diffraction pattern of Form H of the compound of formula I.
Figure 16 shows the Differential Scanning Calorimetry and Thermogravimetric Analysis of Form H of the compound of formula I.
Figure 17 shows the X-ray powder diffraction pattern of Form I of the compound of formula I.
Figure 18 shows the Differential Scanning Calorimetry and Thermogravimetric Analysis of Form I of the compound of formula I.
Figure 19 shows the X-ray powder diffraction pattern of Form J of the compound of formula I.
Figure 20 shows the Differential Scanning Calorimetry and Thermogravimetric Analysis of Form J of the compound of formula I.
Figure 21 shows the X-ray powder diffraction pattern of Form K of the compound of formula I.
Figure 22 shows the Differential Scanning Calorimetry and Thermogravimetric Analysis of Form K of the compound of formula I.
Figure 23 shows the X-ray powder diffraction pattern of Form L of the compound of formula I.
Figure 24 shows the Differential Scanning Calorimetry and Thermogravimetric Analysis of Form L of the compound of formula I.
Figure 25 shows the X-ray powder diffraction pattern of Form M of the compound of formula I.
Figure 26 shows the Differential Scanning Calorimetry and Thermogravimetric Analysis of Form M of the compound of formula I.
Figure 27 shows the X-ray powder diffraction pattern of Form N of the compound of formula I.
Figure 28 shows the Differential Scanning Calorimetry and Thermogravimetric Analysis of Form N of the compound of formula I.
Figure 29 shows the X-ray powder diffraction pattern of Form O of the compound of formula I.
Figure 30 shows the Differential Scanning Calorimetry and Thermogravimetric Analysis of Form O of the compound of formula I.
Figure 31 shows the X-ray powder diffraction pattern of Form P of the compound of formula I.
Figure 32 shows the Differential Scanning Calorimetry and Thermogravimetric Analysis of Form P of the compound of formula I.
Figure 33 shows the X-ray powder diffraction pattern of Form Q of the compound of formula I.
Figure 34 shows the Differential Scanning Calorimetry and Thermogravimetric Analysis of Form Q of the compound of formula I.
Figure 35 shows the X-ray powder diffraction pattern of Form R of the compound of formula I.
Figure 36 shows the Differential Scanning Calorimetry and Thermogravimetric Analysis of Form R of the compound of formula I.
Figure 37 shows the X-ray powder diffraction pattern of Form S of the compound of formula I.
Figure 38 shows the Differential Scanning Calorimetry and Thermogravimetric Analysis of Form S of the compound of formula I.
Figure 39 shows the X-ray powder diffraction pattern of Form T of the compound of formula I.
Figure 40 shows the Differential Scanning Calorimetry and Thermogravimetric Analysis of Form T of the compound of formula I.
Figure 41 shows the X-ray powder diffraction pattern of Form U of the compound of formula I.
Figure 42 shows the Differential Scanning Calorimetry and Thermogravimetric Analysis of Form U of the compound of formula I.
Figure 43 shows the X-ray powder diffraction pattern of Form V of the compound of formula I.
Figure 44 shows the Differential Scanning Calorimetry and Thermogravimetric Analysis of Form V of the compound of formula I.
Figure 45 shows the X-ray powder diffraction pattern of Form W of the compound of formula I.
Figure 46 shows the X-ray powder diffraction pattern of Form X of the compound of formula I.
Figure 47 shows the X-ray powder diffraction pattern of Form Y of the compound of formula I.
Figure 48 shows the X-ray powder diffraction pattern of Form Z of the compound of formula I.
Figure 49 shows the Differential Scanning Calorimetry and Thermogravimetric Analysis of Form Z of the compound of formula I.
Figure 50 shows the DVS diagram of Form C.
Figure 51 shows the X-ray powder diffraction pattern of Form C for stability study.

### Detailed Description of Embodiments

The content of the present invention is described in detail with the following examples. If a specific condition is not indicated in the examples, a conventional condition is used in an experimental method. The listed examples are intended to better illustrate the content of the present invention, but should not be construed as limiting the content of the present invention. According to the above-mentioned content of the invention, those skilled in the art can make unsubstantial modifications and adjustments to the embodiments, which still fall within the protection scope of the present invention.

The structures of the compounds are determined by nuclear magnetic resonance (NMR) or (and) mass spectrometry (MS). The NMR shift (δ) is given in the unit of 10-6 (ppm). NMR is determined with Bruker Avance III 400 and Bruker Avance 300 NMR spectrometer; the solvents for determination are deuterated dimethyl sulfoxide (DMSO-d6), deuterated chloroform (CDCl3) and deuterated methanol (CD3OD); and the internal standard is tetramethylsilane (TMS).

MS is determined with Agilent 6120B (ESI) and Agilent 6120B (APCI).

HPLC is measured with Agilent 1260DAD high pressure liquid chromatography (Zorbax SB-C18 100 × 4.6 mm).

XRPD is determined using an X-ray powder diffractometer, Bruker D8 Advance (Bruker, GER). The 2θ scan angle is from 3° to 45°, the scan step size is 0.02°, and the exposure time is 0.12 seconds. For the test of the sample, the tube voltage and current are 40 kV and 40 mA, respectively, and the sample pan is a zero background sample pan.

The TGA is determined using TA Model TA Discovery 55 (TA, US). 2-5 mg of a sample is placed in a balanced, open aluminium sample pan and automatically weighed in a TGA furnace. The sample is heated to the final temperature at a rate of 10°C/min with a nitrogen purge rate at 60 mL/min at the sample and 40 mL/min at the balance.

The DSC is determined using TA Model TA Discovery 2500 (TA, US). A 1-2 mg sample is accurately weighed and placed in a perforated DSC Tzero sample pan and heated to the final temperature at a rate of 10°C/min with a nitrogen purge rate in the furnace at 50 mL/min.

The known starting materials of the present invention can be synthesised by or according to methods known in the art (e.g., the method in the patent WO 2019240938 A1), or can be purchased from Titan Technology Co., Ltd., Energy Chemical Co., Ltd., Shanghai Demo Co., Ltd., Chengdu Kelong Chemical Co., Ltd., Accela ChemBio Co., Ltd., J&K Scientific Co., Ltd. and other companies.

### Example 1 Preparation of crude free base of compound I

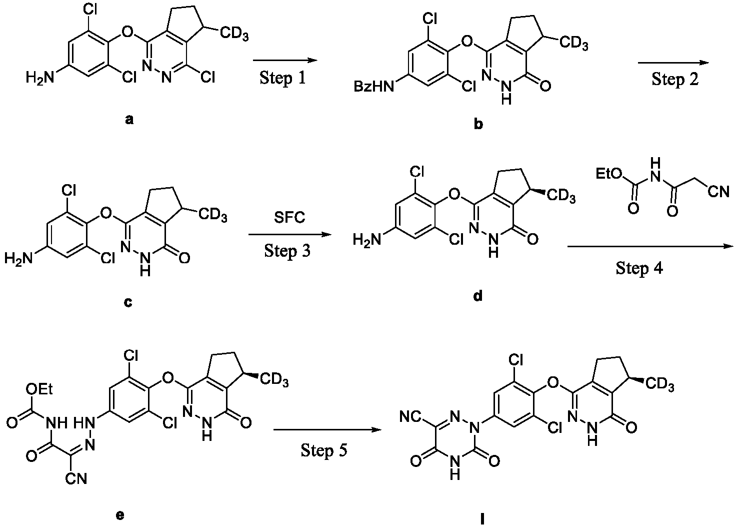

### Step 1: preparation of compound b

### N-(3,5-dichloro-4-((7-(methyl-d3)-1-oxo-2,5,6,7-tetrahydro-1H-cyclopenta[d]pyridazin-4-yl)oxy)phenyl)benzamid

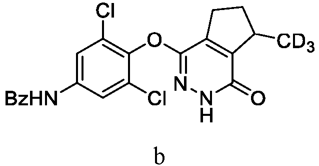

4.62 kg of compound a was completely dissolved in 25.0 L of glacial acetic acid and added to a 100 L reaction kettle, 3300 g of benzoic anhydride was added, and the mixture was reacted at room temperature for about 4 hours with stirring turned on. The reaction was monitored by TLC (n-hexane/ethyl acetate = 5/1) until compound a disappeared, then 2722 g of anhydrous sodium acetate was additionally added and the temperature was increased to 120°C for a reaction under stirring for about 18 hours.

The reaction solution was cooled to 60-65°C, and concentrated under reduced pressure to remove most of the acetic acid. After the concentration was completed, 10 L of anhydrous ethanol was added to the residue and uniformly mixed. Then the mixed solution was slowly added to 250 L of water, while maintaining the rapid stirring, and a large amount of solid precipitated during the addition, and stirring was continued for about 0.5 hours after the addition, followed by centrifugation. The filter cake was washed with purified water (20 L × 2) to give compound b in 100% yield, which went directly to the next step.

1H NMR (400 MHz, DMSO) δ 12.07(s, 1H), 10.55(s, 1H), 8.04(s, 2H), 7.98-7.95(m, 2H), 7.63-7.50(m, 3H), 3.29-3.25 (m, 1H), 3.02-2.90 (m, 2H), 2.39-2.36 (m, 1H), 1.75-1.72 (m, 1H).

LCMS m/z =433.1 [M+1]+

### Step 2: preparation of compound c

### 4-(4-amino-2,6-dichlorophenoxy)-7-(methyl-d3)-2,5,6,7-tetrahydro-1H-cyclopenta[d]pyridazin-1-one

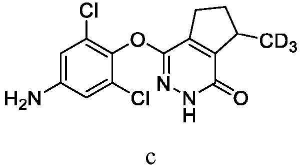

To a 100 L reaction kettle, 5.76 kg of the crude compound b from the previous step, a potassium hydroxide solution (2606 g KOH dissolved in 19.5 L of purified water) and 6.0 L of anhydrous ethanol were added under stirring. After the complete addition, the mixture was heated to reflux and reacted for about 16 hours, and the raw material was controlled for a complete reaction.

The temperature was reduced to 25°C, 30 L of water was added, the pH was adjusted to 8-9 with an ammonium chloride solid, and 35.0 L of ethyl acetate was added and stirred. The solution was phase-separated. The aqueous phase was extracted with ethyl acetate (15.0 L × 2). The organic phases were combined and washed with a 5% aqueous sodium chloride solution (25 L × 2). The organic phase was dried over 3.0 Kg of anhydrous sodium sulphate, filtered, and concentrated until no significant distillate flowed out, so as to obtain a crude product.

The crude product and 7.0 L of an aqueous 10% dioxane solution were heated for complete dissolution, cooled to room temperature, and crystallised with stirring for about 16 hours, followed by filtration to obtain a wet product, which was repeated purified twice and dried under vacuum at 50°C for about 12 hours to give 1507 g of compound c.

1H NMR (400 MHz, DMSO) δ 11.98 (s, 1H), 6.67 (m, 2H), 5.60 (s, 2H), 3.30-3.19 (m, 1H), 3.03-2.93 (m, 1H), 2.90-2.70 (m, 1H), 2.35 (m,1H), 1.69 (m, 1H).

LCMS m/z =329.0 [M+1]+

### Step 3: preparation of compound d

### (R)-4-(4-amino-2,6-dichlorophenoxy)-7-(methyl-d3)-2,5,6,7-tetrahydro-1H-cyclopenta[d]pyridazin-1-one

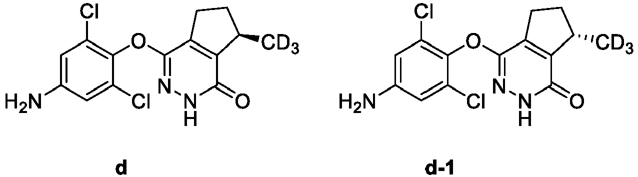

3298 g of racemate c was subjected to chiral resolution to give, two optical isomers from separation:
Compound d (retention time: 1.583 min, 1230 g, off-white solid, ee% = 99.60%, yield 37.3%); and
compound d-1 (retention time: 1.926 min, 1255 g, off-white solid, ee% = 99.76%, yield 38.1%).

Resolution conditions:
Instrument: MG III preparative SFC; column: Whelk O1(S, S), 300 × 50 mm I.D., 10 µm;
mobile phase: A: CO₂, B: methanol; gradient: B 40%; flow rate: 200 mL/min; back pressure: 100 bar;
column temperature: 38°C; wavelength: 220 nm; period: 4.5 min; sample preparation: the racemate was dissolved in methanol/dichloromethane to achieve 50 mg/ml; and injection: 17 ml/injection.

### Compound d

1H NMR (400 MHz, DMSO) δ 11.98 (s, 1H), 6.67 (s, 2H), 5.60 (s, 2H), 3.30-3.19 (m, 1H), 3.03-2.93 (m, 1H), 2.90-2.70 (m, 1H), 2.35 (dtd,1H), 1.69 (ddt, 1H).

LCMS m/z =329.1 [M+1]+

### Compound d-1

1H NMR (400 MHz, DMSO) δ 11.98 (s, 1H), 6.68 (d,2H), 5.60 (s, 2H), 3.29-3.18 (m, 1H), 2.97 (tdd, 1H), 2.90-2.72 (m, 1H), 2.35 (dtd, 1H), 1.69 (ddt, 1H).

LCMS m/z =329.0 [M+1]+

### Step 4: preparation of compound e

### Ethyl (R,Z)-(2-cyano-2-(2-(3,5-dichloro-4-((7-(methyl-d3)-1-oxo-2,5,6,7-tetrahydro-1H-cyclopenta[d]pyridazin-4-yl)oxy)phenyl)hydrazineylidene)acetyl)carbamate

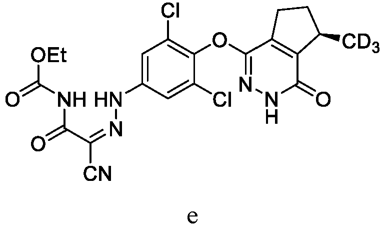

To a 100 L reaction kettle, 16.0 kg of acetic acid, 4.0 kg of purified water and 2.0 kg of compound d were added with stirring. The temperature was reduced to 0 ± 5°C, then 2.36 kg of hydrochloric acid was added, and after the addition, the temperature was maintained at 0 ± 5°C with stirring for about 20 minutes. A sodium nitrite solution (0.5 kg of sodium nitrite dissolved in 1.0 kg of purified water) was dropwise added with the temperature being controlled at 0 ± 5°C, and after the addition, the temperature was maintained at 0 ± 5°C for reaction for 2 hours. The temperature was controlled at 5 ± 5°C and a sodium acetate solution (1.5 kg of sodium acetate dissolved in 6.0 kg of purified water) was added dropwise, then 0.99 kg of N-cyanoacetourethane was added, and then the temperature was increased to 10 ± 5°C for a reaction for about 2 hours. Then a sample was taken for HPLC monitoring, after which time samples were taken at each about 2-hour interval, and the reaction was not stopped until the content of compound d was determined by HPLC to be ≤ 1.0%.

After the completion of the reaction, the temperature was controlled to 10 ± 5°C, and 30.0 kg of purified water was added to the reaction kettle. After the addition, the temperature was controlled at 10 ± 5°C with stirring continued for 1 hour, followed by filtration, and the cake was washed with 3.0 kg of purified water. The filter cake and 12.6 kg of anhydrous ethanol were added to a 100 L reaction kettle, heated to 50 ± 5°C, and stirred for about 1 hour. The mixture was cooled to 20 ± 5°C, stirred for 0.5 hours and filtered, and the filter cake was washed once with 1.26 kg of anhydrous ethanol.

The filter cake was dried at 55 ± 5°C with vacuum ≤ -0.07 MPa for about 17 hours, and compound e was obtained and collected, weighing 2.6327 kg.

1H NMR (400 MHz, DMSO) δ 12.08 (d, 2H), 10.88 (s, 1H), 7.99 (s, 2H), 4.21 (q, 2H), 3.30-3.17 (m, 1H), 3.08-2.95 (m, 1H), 2.95-2.80 (m, 1H), 2.38 (ddd, 1H), 1.78-1.63 (m, 1H), 1.28 (t, 3H).

LCMS m/z =496.1 [M+1]+

### Step 5: preparation of compound of formula I

### (R)-2-(3,5-dichloro-4-((7-(methyl-d3)-1-oxo-2,5,6,7-tetrahydro-1H-cyclopenta[d]pyridazin-4-yl)oxy)phenyl)-3,5-dioxo-2,3,4,5-tetrahydro-1,2,4-triazine-6-carbonitrile

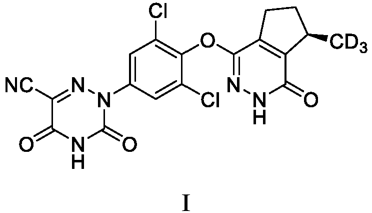

To a 100 L reaction kettle, 12.40 kg of N,N-dimethylacetamide, 2.6269 kg of compound e and 0.54 kg of sodium acetate were added with stirring. After the addition, the temperature was increased and the internal temperature was maintained at 115 ± 5°C for a reaction for about 2 hours. Then a sample was taken for HPLC monitoring, after which time samples were taken at each about 2-hour interval, and the reaction was not stopped until the content of compound e was determined by HPLC to be ≤ 1.0%.

After the completion of the reaction, the temperature was reduced to 60 ± 5°C, 0.788 kg of purified water was added to the reaction solution, and after the addition, the reaction solution was filtered while still hot and quickly added to 13.66 kg of purified water, and the temperature was lowered to 10 ± 5°C. After filtration, the filter cake was added to 20 L of dimethyl sulfoxide and warmed for complete dissolution. 800 L of acetone was added and stirred for 0.5 to 1 h, and then filtered. The filter cake was dried at 55 ± 5°C with vacuum ≤ -0.07 MPa for about 20 hours to give the amorphous form of the compound of formula (I), weighing 1.56 kg.

1H NMR (400 MHz, DMSO) δ 13.26 (s, 1H), 12.09 (s, 1H), 7.79 (s, 2H), 3.32-3.24 (m, 1H), 3.10-2.99 (m, 1H), 2.96-2.88 (m, 1H), 2.45-2.31 (m, 1H), 1.77-1.69 (m, 1H).

LCMS m/z = 450.0 [M+1]+.

### Example 2 Preparation of Form A of compound of formula I

To 200 mg of the compound of formula I prepared in Example 1, 10 ml of water was added, stirred at room temperature for 3-5 h, and filtered to give Form A. The Form A of the compound of formula I was characterised by XRPD, DSC and TGA, with an XRPD pattern shown in Figure 1 and DSC and TGA diagram shown in Figure 2. TGA results show that Form A has a weight loss of 7.4% during heating to 130°C and decomposes at 315°C or more. DSC results show that Form A has an endothermic signal corresponding to a weight loss of TGA at around 99°C and a crystal transformation exothermic peak at around 171°C. The results of the thermal crystal transformation experiments show that Form A is still Form A when heated to 150°C and transformed to Form C when heated to 300°C. NMR results show that the sample has no solvent residue. The water content test results show that Form A has a water content of 8.2%, which corresponds to the weight loss of TGA. In summary, Form A is a dihydrate.

The XRPD diffraction peaks for Form A are listed in the table below:

| Index | Angle(°) | d Value (Å) | Rel.Intensity |
|---|---|---|---|
| 1 | 5.68 | 15.56011 | 100.0 |
| 2 | 10.62 | 8.36035 | 9.0 |
| 3 | 11.44 | 7.77066 | 87.4 |
| 4 | 12.00 | 7.41061 | 7.9 |
| 5 | 12.68 | 7.01902 | 16.2 |
| 6 | 13.26 | 6.71560 | 7.4 |
| 7 | 14.56 | 6.12646 | 11.2 |
| 8 | 15.56 | 5.74481 | 5.6 |
| 9 | 16.02 | 5.58175 | 11.5 |
| 10 | 16.47 | 5.43427 | 5.6 |
| 11 | 17.19 | 5.21335 | 51.3 |
| 12 | 18.02 | 4.97910 | 5.6 |
| 13 | 19.44 | 4.62831 | 12.3 |
| 14 | 19.79 | 4.54970 | 12.3 |
| 15 | 20.12 | 4.47802 | 9.3 |
| 16 | 20.30 | 4.44104 | 8.6 |
| 17 | 21.13 | 4.27299 | 7.9 |
| 18 | 22.11 | 4.09394 | 5.6 |
| 19 | 22.47 | 4.03008 | 6.5 |
| 20 | 22.79 | 3.97795 | 10.7 |
| 21 | 23.27 | 3.89937 | 13.8 |
| 22 | 24.94 | 3.65316 | 19.6 |
| 23 | 25.55 | 3.57258 | 12.4 |
| 24 | 25.82 | 3.53747 | 12.0 |
| 25 | 26.40 | 3.46477 | 26.2 |
| 26 | 26.93 | 3.40215 | 8.8 |
| 27 | 27.16 | 3.37512 | 8.3 |
| 28 | 27.57 | 3.32898 | 8.0 |
| 29 | 27.78 | 3.30538 | 7.4 |
| 30 | 28.91 | 3.18698 | 9.5 |
| 31 | 29.47 | 3.13135 | 17.9 |
| 32 | 30.35 | 3.04934 | 9.6 |
| 33 | 31.82 | 2.92167 | 5.8 |
| 34 | 32.45 | 2.87161 | 5.0 |
| 35 | 33.18 | 2.81477 | 8.7 |
| 36 | 34.08 | 2.74950 | 9.1 |
| 37 | 34.82 | 2.69831 | 4.3 |
| 38 | 35.89 | 2.62826 | 11.8 |
| 39 | 36.10 | 2.61480 | 10.6 |
| 40 | 36.93 | 2.56379 | 4.2 |
| 41 | 38.02 | 2.50104 | 5.1 |
| 42 | 40.18 | 2.38778 | 4.2 |
| 43 | 40.96 | 2.35024 | 5.0 |
| 44 | 41.41 | 2.32937 | 11.6 |
| 45 | 43.27 | 2.24757 | 4.0 |

### Example 3 Preparation of Form B of compound of formula I

To 150 mg of the compound of formula I prepared in Example 1, 4-methyl-2-pentanone was added until completely dissolved. n-Hexane was added dropwise until a solid precipitated, stirred for 3-5 h and filtered, and the resulting solid was dried overnight at room temperature to give Form B. Form B of the compound of formula I was characterised by XRPD, DSC and TGA. The XRPD pattern is shown in Figure 3, and the DSC and TGA diagram is shown in Figure 4. TGA results show that Form B has a weight loss of 8.8% during heating to 200°C and may decompose at 315°C or more. DSC results show that Form B has an endothermic signal corresponding to a weight loss at around 139°C. NMR results show no change in the structure of the compound, solvent peaks visible at 1.40 ppm for cyclohexane, and solvent peaks visible at 0.85 ppm, 1.99 ppm, 2.06 ppm, and 2.30 ppm for 4-methyl-2-pentanone. Based on the integration results, the ratio of the compound to cyclohexane is 1 : 0.05, the ratio of the compound to 4-methyl-2-pentanone is 1 : 0.4, and the roughly estimated mass percentage of 4-methyl-2-pentanone is about 8.5%, corresponding to the weight loss of TGA. In summary, Form B is a solvate of 4-methyl-2-pentanone in a ratio of 1 : 0.4 of the compound to 4-methyl-2-pentanone.

The XRPD diffraction peaks for Form B are listed in the table below:

| Index | Angle(°) | d Value (Å) | Rel.Intensity |
|---|---|---|---|
| 1 | 8.03 | 11.02329 | 100.0 |
| 2 | 10.00 | 8.87468 | 7.3 |
| 3 | 11.05 | 8.04052 | 16.0 |
| 4 | 11.82 | 7.51865 | 22.4 |
| 5 | 14.12 | 6.31632 | 12.2 |
| 6 | 14.97 | 5.96311 | 12.3 |
| 7 | 15.67 | 5.70312 | 8.3 |
| 8 | 16.14 | 5.54248 | 16.8 |
| 9 | 16.55 | 5.40945 | 12.6 |
| 10 | 17.64 | 5.08524 | 9.1 |
| 11 | 18.67 | 4.81379 | 7.4 |
| 12 | 19.56 | 4.60179 | 6.3 |
| 13 | 20.22 | 4.45739 | 8.1 |
| 14 | 20.65 | 4.36899 | 6.8 |
| 15 | 22.01 | 4.11112 | 34.4 |
| 16 | 22.34 | 4.05335 | 20.3 |
| 17 | 23.43 | 3.87493 | 16.0 |
| 18 | 24.34 | 3.73788 | 14.8 |
| 19 | 25.18 | 3.62149 | 25.1 |
| 20 | 27.63 | 3.32251 | 8.0 |
| 21 | 29.30 | 3.14838 | 12.2 |
| 22 | 31.24 | 2.97053 | 7.3 |
| 23 | 32.68 | 2.85336 | 7.6 |
| 24 | 36.59 | 2.58483 | 6.2 |

### Example 4 Preparation of Form C of compound of formula I

To about 150 mg of the compound of formula I prepared in Example 1, 3.0 mL of chloroform was added, stirred at 50°C for 1 day and centrifuged, and the resulting solid was dried under vacuum overnight at room temperature to give Form C. Form C of the compound of formula I was characterised by XRPD, DSC and TGA, with an XRPD pattern shown in Figure 5 and DSC and TGA diagram shown in Figure 6. TGA results show that Form C has a weight loss of 0.8% during heating to 200°C and may decompose at 315°C or more. DSC results show that Form C has an endothermic signal corresponding to a weight loss of TGA at around 70°C and no melting endothermic signal at 300°C or less. In summary, Form C is a crystal form free of water.

The XRPD diffraction peaks for Form C are listed in the table below:

| Index | Angle(°) | d Value (Å) | Rel.Intensity |
|---|---|---|---|
| 1 | 9.80 | 9.04888 | 11.2 |
| 2 | 11.03 | 8.05452 | 51.0 |
| 3 | 11.38 | 7.80995 | 12.7 |
| 4 | 11.71 | 7.59248 | 19.7 |
| 5 | 14.12 | 6.31632 | 42.2 |
| 6 | 15.79 | 5.66206 | 59.5 |
| 7 | 16.45 | 5.44052 | 9.6 |
| 8 | 16.97 | 5.27704 | 100.0 |
| 9 | 17.77 | 5.04755 | 12.7 |
| 10 | 18.74 | 4.79454 | 12.2 |
| 11 | 19.33 | 4.65517 | 10.8 |
| 12 | 19.73 | 4.56260 | 40.1 |
| 13 | 21.33 | 4.23584 | 14.1 |
| 14 | 22.51 | 4.02348 | 38.0 |
| 15 | 22.79 | 3.97795 | 19.2 |
| 16 | 23.27 | 3.89937 | 87.9 |
| 17 | 23.76 | 3.82410 | 58.9 |
| 18 | 24.55 | 3.70732 | 15.3 |
| 19 | 24.90 | 3.65850 | 10.8 |
| 20 | 25.37 | 3.59557 | 37.4 |
| 21 | 26.36 | 3.46952 | 11.6 |
| 22 | 26.69 | 3.42966 | 11.7 |
| 23 | 27.61 | 3.32467 | 74.0 |
| 24 | 28.21 | 3.25930 | 30.7 |
| 25 | 28.52 | 3.22670 | 22.9 |
| 26 | 28.93 | 3.18503 | 17.5 |
| 27 | 29.78 | 3.10161 | 11.9 |
| 28 | 30.72 | 3.01620 | 8.4 |
| 29 | 31.12 | 2.98054 | 9.2 |
| 30 | 31.49 | 2.94912 | 8.4 |
| 31 | 32.19 | 2.89169 | 12.1 |
| 32 | 32.52 | 2.86549 | 10.5 |
| 33 | 32.97 | 2.83094 | 12.7 |
| 34 | 33.73 | 2.77460 | 36.8 |
| 35 | 35.92 | 2.62580 | 12.0 |
| 36 | 36.41 | 2.59552 | 9.2 |
| 37 | 36.72 | 2.57659 | 8.5 |
| 38 | 37.25 | 2.54545 | 10.2 |
| 39 | 37.85 | 2.51086 | 13.6 |
| 40 | 39.25 | 2.43505 | 7.7 |
| 41 | 39.87 | 2.40326 | 7.1 |
| 42 | 41.74 | 2.31428 | 6.3 |
| 43 | 42.28 | 2.29000 | 7.3 |
| 44 | 43.37 | 2.24353 | 8.1 |
| 45 | 43.52 | 2.23712 | 7.3 |

### Example 5 Preparation of Form D of compound of formula I

To 20 mg of the compound of formula I prepared in Example 1, ethylene glycol monomethyl ether was added until completely dissolved. Toluene was added dropwise until a solid precipitated, stirred for 3-5 hours and centrifuged, and the resulting solid was dried under vacuum overnight at room temperature to give Form D. Form D of the compound of formula I was characterised by XRPD, DSC and TGA, with an XRPD pattern shown in Figure 7 and DSC and TGA diagram shown in Figure 8. TGA results show that Form D has a weight loss of 7.6% during heating to 150°C, and a weight loss of 8.5% at 150-200°C, and may decompose at 330°C or more. DSC results show that Form D has endothermic peaks corresponding to weight losses at around 139°C and 177°C. NMR results show no change in the structure of the compound, and solvent peaks visible at 2.30 ppm, 7.18 ppm, and 7.24 ppm for toluene. Based on the integration results, the roughly estimated mass percentage is about 14.0%, corresponding to the weight loss of TGA. In summary, Form D is a solvate of toluene in a ratio of 1 : 0.4 of the compound to toluene.

The XRPD diffraction peaks for Form D are listed in the table below:

| Index | Angle(°) | d Value (Å) | Rel.Intensity |
|---|---|---|---|
| 1 | 5.93 | 14.89987 | 100.0 |
| 2 | 10.83 | 8.19726 | 8.2 |
| 3 | 11.14 | 7.97128 | 7.2 |
| 4 | 11.90 | 7.47024 | 59.5 |
| 5 | 12.97 | 6.86387 | 10.2 |
| 6 | 13.38 | 6.65812 | 16.7 |
| 7 | 15.50 | 5.76589 | 59.1 |
| 8 | 16.59 | 5.39713 | 8.2 |
| 9 | 16.84 | 5.31843 | 15.7 |
| 10 | 17.91 | 5.01045 | 32.1 |
| 11 | 18.51 | 4.85279 | 13.1 |
| 12 | 18.90 | 4.75651 | 7.5 |
| 13 | 19.64 | 4.58429 | 7.2 |
| 14 | 20.24 | 4.45329 | 13.2 |
| 15 | 20.86 | 4.32619 | 53.6 |
| 16 | 21.79 | 4.14945 | 12.4 |
| 17 | 22.46 | 4.03339 | 7.6 |
| 18 | 23.02 | 3.93980 | 9.5 |
| 19 | 23.49 | 3.86585 | 11.1 |
| 20 | 23.95 | 3.79488 | 53.6 |
| 21 | 25.31 | 3.60330 | 8.3 |
| 22 | 26.25 | 3.48383 | 24.3 |
| 23 | 26.58 | 3.44361 | 6.6 |
| 24 | 27.14 | 3.37736 | 7.2 |
| 25 | 28.95 | 3.18307 | 8.3 |
| 26 | 29.94 | 3.08698 | 13.9 |
| 27 | 31.26 | 2.96887 | 5.6 |
| 28 | 32.48 | 2.86855 | 26.2 |
| 29 | 32.74 | 2.84884 | 9.6 |
| 30 | 33.26 | 2.80894 | 6.8 |
| 31 | 34.58 | 2.71422 | 5.5 |
| 32 | 35.92 | 2.62580 | 8.0 |
| 33 | 36.29 | 2.60270 | 18.7 |
| 34 | 42.61 | 2.27560 | 5.9 |

### Example 6 Preparation of Form E of compound of formula I

To 55 mg of the compound of formula I prepared in Example 1, N,N-dimethylformamide (DMF) was added until completely dissolved. Water was added dropwise until a solid precipitated, stirred for 3-5 h and centrifuged, and the resulting solid was dried under vacuum overnight at room temperature to give Form E. Form E of the compound of formula I was characterised by XRPD, DSC and TGA, with an XRPD pattern shown in Figure 9 and DSC and TGA diagram shown in Figure 10. TGA results show that Form E has a weight loss of 10.2% during heating to 120°C, and a weight loss of 11.2% at 120-180°C, and may decompose at 330°C or more. DSC results show that Form E has endothermic peaks at around 112°C and 157°C corresponding to weight losses of TGA. The results of the thermal crystal transformation experiments show that Form E transforms to Form C by desolvation. NMR results show no change in the structure of the compound, and solvent peaks visible at 2.73 ppm, 2.89 ppm, and 7.95 ppm for DMF. Based on the integration results, the roughly estimated mass percentage is about 20.5%, corresponding to the weight loss of TGA. In summary, Form E is a solvate of DMF in a ratio of 1 : 0.8 of the compound to DMF.

The XRPD diffraction peaks for Form E are listed in the table below:

| Index | Angle(°) | d Value (Å) | Rel.Intensity |
|---|---|---|---|
| 1 | 4.75 | 18.60748 | 79.2 |
| 2 | 5.39 | 16.39893 | 16.9 |
| 3 | 5.76 | 15.35077 | 19.3 |
| 4 | 8.23 | 10.76464 | 17.3 |
| 5 | 9.47 | 9.36149 | 100.0 |
| 6 | 10.83 | 8.19726 | 14.0 |
| 7 | 11.49 | 7.73176 | 11.9 |
| 8 | 13.15 | 6.77411 | 4.9 |
| 9 | 14.21 | 6.27402 | 48.8 |
| 10 | 14.66 | 6.08674 | 6.1 |
| 11 | 15.83 | 5.64851 | 5.7 |
| 12 | 16.43 | 5.44678 | 13.0 |
| 13 | 17.34 | 5.16803 | 5.3 |
| 14 | 17.73 | 5.05826 | 5.0 |
| 15 | 17.95 | 4.99995 | 6.6 |
| 16 | 18.43 | 4.87255 | 14.7 |
| 17 | 18.70 | 4.80414 | 13.4 |
| 18 | 19.02 | 4.72841 | 13.3 |
| 19 | 20.06 | 4.49049 | 12.4 |
| 20 | 20.38 | 4.42481 | 12.3 |
| 21 | 20.63 | 4.37293 | 6.9 |
| 22 | 21.21 | 4.25804 | 4.9 |
| 23 | 21.60 | 4.18498 | 8.7 |
| 24 | 22.84 | 3.96834 | 26.5 |
| 25 | 23.54 | 3.85682 | 7.5 |
| 26 | 23.80 | 3.81822 | 4.8 |
| 27 | 24.79 | 3.67461 | 7.3 |
| 28 | 25.23 | 3.61367 | 5.8 |
| 29 | 25.41 | 3.59043 | 8.1 |
| 30 | 26.11 | 3.50070 | 5.5 |
| 31 | 26.42 | 3.46241 | 15.3 |
| 32 | 27.63 | 3.32251 | 6.6 |
| 33 | 28.67 | 3.21068 | 10.7 |
| 34 | 28.97 | 3.18112 | 7.4 |
| 35 | 29.14 | 3.16369 | 6.7 |
| 36 | 29.55 | 3.12385 | 5.7 |
| 37 | 29.78 | 3.10161 | 5.7 |
| 38 | 32.11 | 2.89794 | 8.8 |
| 39 | 33.51 | 2.79021 | 5.3 |
| 40 | 36.59 | 2.58483 | 5.6 |
| 41 | 36.95 | 2.56263 | 5.4 |
| 42 | 43.64 | 2.23234 | 6.2 |

### Example 7 Preparation of Form F of compound of formula I

To 20 mg of the compound of formula I prepared in Example 1, ethylene glycol methyl ether was added until completely dissolved. Acetone was added dropwise until a solid precipitated, stirred for 3-5 h and centrifuged, and the resulting solid was dried under vacuum overnight at room temperature to give Form F. Form F of the compound of formula I was characterised by XRPD, DSC and TGA, with an XRPD pattern shown in Figure 11 and DSC and TGA diagram shown in Figure 12. TGA results show that Form F has a weight loss of 11.3% during heating to 150°C and may decompose at 330°C or more. DSC results show that Form F has an endothermic signal corresponding to a weight loss of TGA at around 125-180°C and a crystal transformation exothermic peak at around 204°C. The results of the thermal crystal transformation experiments show that Form F transforms to Form C when heated to 230°C or more. NMR results show no change in the structure of the compound, and a solvent peak visible at 2.09 ppm for acetone. Based on the integration results, the roughly estimated mass percentage is about 10.0%, corresponding to the weight loss of TGA. In summary, Form F is a solvate of acetone in a ratio of 1 : 1 of the compound to acetone.

The XRPD diffraction peaks for Form F are listed in the table below:

| Index | Angle(°) | d Value (Å) | Rel.Intensity |
|---|---|---|---|
| 1 | 6.67 | 13.25713 | 45.1 |
| 2 | 7.65 | 11.58018 | 72.7 |
| 3 | 8.34 | 10.61526 | 57.0 |
| 4 | 10.17 | 8.72364 | 26.1 |
| 5 | 12.37 | 7.19265 | 16.5 |
| 6 | 13.42 | 6.63918 | 39.2 |
| 7 | 15.48 | 5.77296 | 49.1 |
| 8 | 16.12 | 5.54899 | 5.9 |
| 9 | 16.49 | 5.42805 | 5.3 |
| 10 | 16.80 | 5.33038 | 5.8 |
| 11 | 17.48 | 5.12905 | 10.4 |
| 12 | 18.02 | 4.97910 | 8.1 |
| 13 | 18.49 | 4.85772 | 100.0 |
| 14 | 19.05 | 4.71912 | 15.3 |
| 15 | 20.20 | 4.46150 | 9.6 |
| 16 | 20.47 | 4.40470 | 23.0 |
| 17 | 21.06 | 4.28804 | 9.4 |
| 18 | 21.64 | 4.17782 | 16.1 |
| 19 | 22.38 | 4.04668 | 13.5 |
| 20 | 22.86 | 3.96515 | 16.2 |
| 21 | 23.17 | 3.91481 | 19.2 |
| 22 | 23.41 | 3.87797 | 27.2 |
| 23 | 23.66 | 3.83890 | 12.3 |
| 24 | 24.20 | 3.75761 | 9.6 |
| 25 | 24.59 | 3.70183 | 9.1 |
| 26 | 24.88 | 3.66117 | 17.9 |
| 27 | 26.05 | 3.50799 | 11.8 |
| 28 | 26.46 | 3.45769 | 8.4 |
| 29 | 26.91 | 3.40442 | 10.5 |
| 30 | 27.59 | 3.32682 | 8.0 |
| 31 | 28.15 | 3.26550 | 27.1 |
| 32 | 28.75 | 3.20273 | 20.8 |
| 33 | 29.45 | 3.13323 | 5.7 |
| 34 | 30.31 | 3.05288 | 7.6 |
| 35 | 30.54 | 3.03179 | 9.0 |
| 36 | 31.22 | 2.97219 | 6.7 |
| 37 | 31.53 | 2.94586 | 12.7 |
| 38 | 32.46 | 2.87008 | 6.0 |
| 39 | 32.85 | 2.83986 | 13.5 |
| 40 | 33.36 | 2.80170 | 6.9 |
| 41 | 35.59 | 2.64691 | 7.2 |
| 42 | 36.14 | 2.61237 | 10.7 |
| 43 | 37.56 | 2.52744 | 6.4 |
| 44 | 39.11 | 2.44216 | 7.2 |
| 45 | 39.71 | 2.41111 | 5.0 |
| 46 | 39.83 | 2.40522 | 4.1 |
| 47 | 40.80 | 2.35762 | 7.5 |
| 48 | 41.13 | 2.34201 | 10.0 |
| 49 | 41.56 | 2.32223 | 5.6 |
| 50 | 42.44 | 2.28319 | 4.4 |
| 51 | 42.98 | 2.25982 | 4.5 |

### Example 8 Preparation of Form G of compound of formula I

To 20 mg of the compound of formula I prepared in Example 1, ethylene glycol methyl ether was added until completely dissolved. Ethyl acetate was added dropwise until a solid precipitated, stirred for 3-5 h and centrifuged, and the resulting solid was dried under vacuum overnight at room temperature to give Form G. Form G of the compound of formula I was characterised by XRPD, DSC and TGA, with an XRPD pattern shown in Figure 13 and DSC and TGA diagram shown in Figure 14. TGA results show that Form G has a weight loss of 16.2% during heating to 200°C and may decompose at 330°C or more. DSC results show that Form G has an endothermic signal corresponding to a weight loss of TGA at around 152°C. NMR results show no change in the structure of the compound, and solvent peaks visible at 1.16 ppm, 1.99 ppm, and 4.03 ppm for ethyl acetate. Based on the integration results, the roughly estimated mass percentage is about 15.0%, corresponding to the weight loss of TGA. In summary, Form G is a solvate of ethyl acetate in a ratio of 1 : 1 of the compound to ethyl acetate.

The XRPD diffraction peaks for Form G are listed in the table below:

| Index | Angle(°) | d Value (Å) | Rel.Intensity |
|---|---|---|---|
| 1 | 8.34 | 10.61526 | 11.1 |
| 2 | 8.69 | 10.19122 | 22.1 |
| 3 | 9.14 | 9.69681 | 21.7 |
| 4 | 10.39 | 8.54597 | 12.3 |
| 5 | 11.03 | 8.05452 | 16.5 |
| 6 | 11.88 | 7.48228 | 22.2 |
| 7 | 12.12 | 7.34034 | 12.8 |
| 8 | 12.62 | 7.05092 | 13.0 |
| 9 | 13.53 | 6.58303 | 32.1 |
| 10 | 14.74 | 6.05533 | 6.3 |
| 11 | 15.34 | 5.82291 | 9.0 |
| 12 | 16.14 | 5.54248 | 13.6 |
| 13 | 16.66 | 5.37266 | 19.2 |
| 14 | 16.94 | 5.28880 | 28.8 |
| 15 | 17.40 | 5.15125 | 100.0 |
| 16 | 18.70 | 4.80414 | 12.3 |
| 17 | 18.82 | 4.77544 | 14.6 |
| 18 | 19.19 | 4.68691 | 14.8 |
| 19 | 19.44 | 4.62831 | 6.3 |
| 20 | 19.79 | 4.54970 | 12.8 |
| 21 | 20.73 | 4.35332 | 20.8 |
| 22 | 21.21 | 4.25804 | 6.4 |
| 23 | 21.70 | 4.16714 | 11.6 |
| 24 | 22.82 | 3.97154 | 17.8 |
| 25 | 23.08 | 3.93039 | 10.6 |
| 26 | 23.23 | 3.90553 | 11.1 |
| 27 | 23.76 | 3.82410 | 19.4 |
| 28 | 24.15 | 3.76614 | 43.6 |
| 29 | 24.36 | 3.73508 | 24.9 |
| 30 | 24.57 | 3.70457 | 10.6 |
| 31 | 24.79 | 3.67461 | 13.1 |
| 32 | 25.60 | 3.56499 | 51.1 |
| 33 | 26.28 | 3.47904 | 13.7 |
| 34 | 26.58 | 3.44361 | 11.1 |
| 35 | 27.16 | 3.37512 | 32.3 |
| 36 | 27.88 | 3.29478 | 8.7 |
| 37 | 28.44 | 3.23478 | 6.6 |
| 38 | 28.81 | 3.19681 | 9.7 |
| 39 | 28.99 | 3.17917 | 8.8 |
| 40 | 29.41 | 3.13700 | 13.1 |
| 41 | 29.69 | 3.11083 | 12.7 |
| 42 | 30.52 | 3.03354 | 11.6 |
| 43 | 30.87 | 3.00250 | 7.4 |
| 44 | 31.67 | 2.93451 | 8.7 |
| 45 | 32.35 | 2.87929 | 8.9 |
| 46 | 32.54 | 2.86397 | 10.6 |
| 47 | 32.76 | 2.84734 | 10.2 |
| 48 | 33.90 | 2.76198 | 6.3 |
| 49 | 34.23 | 2.73853 | 6.7 |
| 50 | 35.07 | 2.68134 | 6.2 |
| 51 | 35.32 | 2.66462 | 6.2 |
| 52 | 36.06 | 2.61723 | 6.9 |
| 53 | 36.99 | 2.56032 | 5.4 |
| 54 | 37.27 | 2.54431 | 5.2 |
| 55 | 37.63 | 2.52299 | 5.1 |
| 56 | 41.48 | 2.32580 | 4.7 |

### Example 9 Preparation of Form H of compound of formula I

To 55 mg of the compound of formula I prepared in Example 1, dimethyl sulfoxide was added until completely dissolved. Water was added dropwise until a solid precipitated, stirred for 3-5 h and centrifuged, and the resulting solid was dried under vacuum overnight at room temperature to give Form H. Form H of the compound of formula I was characterised by XRPD, DSC and TGA, with an XRPD pattern shown in Figure 15 and DSC and TGA diagram shown in Figure 16. TGA results show that Form H has a weight loss of 15.2% during heating to 210°C and may decompose at 320°C or more. DSC results show that Form H has an endothermic signal corresponding to a weight loss of TGA at around 160-230°C. NMR results show no change in the structure of the compound, and a solvent peak visible at 2.54 ppm for DMSO. Based on the integration results, the roughly estimated mass percentage is about 15.0%, corresponding to the weight loss of TGA. In summary, Form H is a solvate of DMSO in a ratio of 1 : 1 of the compound to DMSO.

The XRPD diffraction peaks for Form H are listed in the table below:

| Index | Angle(°) | d Value (Å) | Rel.Intensity |
|---|---|---|---|
| 1 | 5.53 | 15.99644 | 49.0 |
| 2 | 8.54 | 10.37537 | 37.3 |
| 3 | 11.05 | 8.04052 | 100.0 |
| 4 | 12.68 | 7.01902 | 15.2 |
| 5 | 13.50 | 6.60163 | 14.8 |
| 6 | 14.00 | 6.36787 | 10.3 |
| 7 | 15.40 | 5.80139 | 9.4 |
| 8 | 15.85 | 5.64176 | 12.9 |
| 9 | 16.18 | 5.52952 | 8.1 |
| 10 | 16.59 | 5.39713 | 16.9 |
| 11 | 17.11 | 5.23632 | 8.8 |
| 12 | 17.50 | 5.12353 | 8.2 |
| 13 | 18.16 | 4.94304 | 58.8 |
| 14 | 18.53 | 4.84788 | 8.4 |
| 15 | 20.16 | 4.46974 | 13.4 |
| 16 | 21.04 | 4.29182 | 58.3 |
| 17 | 21.91 | 4.12845 | 34.4 |
| 18 | 22.16 | 4.08372 | 42.4 |
| 19 | 22.75 | 3.98438 | 23.7 |
| 20 | 23.45 | 3.87190 | 20.0 |
| 21 | 23.84 | 3.81235 | 10.1 |
| 22 | 25.70 | 3.55243 | 30.3 |
| 23 | 26.30 | 3.47666 | 15.8 |
| 24 | 26.65 | 3.43430 | 20.4 |
| 25 | 27.22 | 3.36844 | 11.5 |
| 26 | 27.57 | 3.32898 | 21.3 |
| 27 | 28.13 | 3.26757 | 15.3 |
| 28 | 28.93 | 3.18503 | 10.5 |
| 29 | 29.57 | 3.12199 | 9.4 |
| 30 | 31.45 | 2.95239 | 11.1 |
| 31 | 33.49 | 2.79164 | 14.9 |
| 32 | 34.97 | 2.68783 | 8.2 |
| 33 | 36.25 | 2.60511 | 9.6 |
| 34 | 37.09 | 2.55457 | 7.5 |

### Example 10 Preparation of Form I of compound of formula I

To 55 mg of Form A of the compound of formula I, isopropyl acetate was added, stirred at 50°C for 10-15 h and centrifuged, and the resulting solid was dried under vacuum at room temperature overnight, to obtain Form I. Form I of the compound of formula I was characterised by XRPD, DSC and TGA, as shown in Figures 17-18, respectively. TGA results show that Form I has a weight loss of 17.7% during heating to 200°C and may decompose at 315°C or more. DSC results show that Form I has an endothermic peak corresponding to a weight loss of TGA at around 158°C. NMR results show no change in the structure of the compound, and solvent peaks visible at 1.17 ppm, 1.96 ppm, and 4.84 ppm for isopropyl acetate. Based on the integration results, the roughly estimated mass percentage is about 17.6%, corresponding to the weight loss of TGA. In summary, Form I is a solvate of isopropyl acetate in a ratio of 1 : 0.9 of the compound to isopropyl acetate.

The XRPD diffraction peaks for Form I are listed in the table below:

| Index | Angle(°) | d Value (Å) | Rel.Intensity |
|---|---|---|---|
| 1 | 8.36 | 10.59076 | 22.5 |
| 2 | 10.17 | 8.72364 | 17.0 |
| 3 | 11.36 | 7.82315 | 21.4 |
| 4 | 12.02 | 7.39881 | 12.4 |
| 5 | 12.72 | 6.99792 | 22.4 |
| 6 | 13.20 | 6.74472 | 38.7 |
| 7 | 14.64 | 6.09464 | 6.0 |
| 8 | 16.55 | 5.40945 | 33.5 |
| 9 | 16.78 | 5.33638 | 100.0 |
| 10 | 17.13 | 5.23056 | 18.3 |
| 11 | 17.42 | 5.14568 | 8.8 |
| 12 | 18.14 | 4.94815 | 9.8 |
| 13 | 18.92 | 4.75180 | 19.0 |
| 14 | 19.85 | 4.53687 | 24.1 |
| 15 | 20.08 | 4.48633 | 9.0 |
| 16 | 20.38 | 4.42481 | 15.4 |
| 17 | 20.55 | 4.38875 | 12.8 |
| 18 | 22.44 | 4.03670 | 7.6 |
| 19 | 22.63 | 4.00383 | 7.9 |
| 20 | 23.19 | 3.91171 | 11.4 |
| 21 | 23.50 | 3.86284 | 22.4 |
| 22 | 23.97 | 3.79198 | 64.3 |
| 23 | 24.50 | 3.71560 | 31.6 |
| 24 | 24.85 | 3.66654 | 10.0 |
| 25 | 25.60 | 3.56499 | 18.5 |
| 26 | 25.93 | 3.52266 | 8.2 |
| 27 | 26.42 | 3.46241 | 15.1 |
| 28 | 26.79 | 3.41814 | 46.7 |
| 29 | 27.31 | 3.35738 | 11.5 |
| 30 | 27.74 | 3.30964 | 8.4 |
| 31 | 28.11 | 3.26965 | 7.9 |
| 32 | 28.42 | 3.23681 | 6.4 |
| 33 | 28.75 | 3.20273 | 8.6 |
| 34 | 29.32 | 3.14647 | 13.2 |
| 35 | 29.55 | 3.12385 | 13.2 |
| 36 | 30.00 | 3.08154 | 21.4 |
| 37 | 30.73 | 3.01448 | 6.2 |
| 38 | 31.45 | 2.95239 | 13.9 |
| 39 | 32.06 | 2.90265 | 6.9 |
| 40 | 32.39 | 2.87621 | 11.1 |
| 41 | 32.87 | 2.83837 | 13.7 |
| 42 | 33.92 | 2.76059 | 6.6 |
| 43 | 34.93 | 2.69044 | 6.3 |
| 44 | 35.85 | 2.63073 | 6.5 |
| 45 | 36.31 | 2.60150 | 6.3 |
| 46 | 36.74 | 2.57542 | 7.0 |
| 47 | 37.60 | 2.52521 | 4.8 |
| 48 | 40.34 | 2.38014 | 9.0 |
| 49 | 40.59 | 2.36787 | 9.3 |
| 50 | 44.22 | 2.20888 | 6.2 |

### Example 11 Preparation of Form J of compound of formula I

To 55 mg of Form A of the compound of formula I, acetonitrile was added, stirred at 50°C for 10-15 h and centrifuged, and the resulting solid was dried under vacuum at room temperature overnight, to obtain Form J. Form J of the compound of formula I was characterised by XRPD, DSC and TGA, as shown in Figures 19-20, respectively. TGA results show that Form J has a weight loss of 4.1% during heating to 150°C and may decompose at 320°C or more. DSC results show that Form J has an endothermic signal corresponding to a weight loss of TGA at around 133°C and a crystal transformation exothermic signal at around 157°C. The results of the thermal crystal transformation experiments show that Form J transforms to Form C by desolvation. NMR results show no change in the structure of the compound, and a solvent peak visible at 2.07 ppm for acetonitrile. Based on the integration results, the ratio of the compound to acetonitrile is 1 : 0.1, and the roughly estimated mass percentage is about 1.29%.

### Example 12 Preparation of Form K of compound of formula I

To 55 mg of the compound of formula I prepared in Example 1, dioxane was added until completely dissolved. n-Heptane was added dropwise until a solid precipitated, stirred for 3-5 h and centrifuged, and the resulting solid was dried under vacuum overnight at room temperature to give Form K. Form K of the compound of formula I was characterised by XRPD, DSC and TGA, as shown in Figures 21-22, respectively. TGA results show that Form K has a weight loss of 15.6% during heating to 210°C and may decompose at 315°C or more. DSC results show that Form K has an endothermic signal corresponding to a weight loss of TGA at around 185-205°C and a recrystallization exothermic peak at around 254°C. The results of the thermal crystal transformation experiments show that Form K transforms to Form C when heated to 260°C or more. NMR results show no change in the structure of the compound, and a solvent peak visible at 3.57 ppm for dioxane. Based on the integration results, the roughly estimated mass percentage is about 14.3%, corresponding to the weight loss of TGA. In summary, Form K is a solvate of dioxane in a ratio of 1 : 0.9 of the compound to dioxane.

The XRPD diffraction peaks for Form K are listed in the table below:

| Index | Angle(°) | d Value (Å) | Rel.Intensity |
|---|---|---|---|
| 1 | 5.74 | 15.40258 | 16.8 |
| 2 | 7.63 | 11.60951 | 100.0 |
| 3 | 8.34 | 10.61526 | 20.4 |
| 4 | 10.15 | 8.74016 | 6.1 |
| 5 | 11.51 | 7.71889 | 8.3 |
| 6 | 12.35 | 7.20379 | 5.4 |
| 7 | 12.64 | 7.04025 | 3.3 |
| 8 | 15.28 | 5.84459 | 4.4 |
| 9 | 16.08 | 5.56204 | 5.0 |
| 10 | 16.72 | 5.35445 | 3.4 |
| 11 | 17.29 | 5.18493 | 4.3 |
| 12 | 17.42 | 5.14568 | 3.7 |
| 13 | 18.33 | 4.89748 | 3.4 |
| 14 | 18.47 | 4.86265 | 3.0 |
| 15 | 19.00 | 4.73307 | 3.8 |
| 16 | 20.41 | 4.41674 | 3.3 |
| 17 | 22.26 | 4.06679 | 12.9 |
| 18 | 23.04 | 3.93666 | 35.4 |
| 19 | 23.27 | 3.89937 | 19.8 |
| 20 | 24.13 | 3.76899 | 3.5 |
| 21 | 24.85 | 3.66654 | 3.9 |
| 22 | 25.99 | 3.51531 | 4.2 |
| 23 | 26.73 | 3.42504 | 3.4 |
| 24 | 27.41 | 3.34639 | 3.0 |
| 25 | 30.05 | 3.07612 | 4.3 |
| 26 | 31.05 | 2.98725 | 4.1 |
| 27 | 31.63 | 2.93774 | 4.8 |
| 28 | 32.54 | 2.86397 | 4.0 |
| 29 | 35.36 | 2.66208 | 2.5 |
| 30 | 36.72 | 2.57659 | 3.1 |
| 31 | 37.36 | 2.53866 | 2.3 |
| 32 | 38.84 | 2.45654 | 4.5 |
| 33 | 39.48 | 2.42300 | 2.6 |

### Example 13 Preparation of Form L of compound of formula I

To 40 mg of the compound of formula I prepared in Example 1, dioxane was added until completely dissolved. Water was added dropwise until a solid precipitated, stirred for 3-5 h and centrifuged, and the resulting solid was dried under vacuum overnight at room temperature to give Form L. Form L of the compound of formula I was characterised by XRPD, DSC and TGA, as shown in Figures 23-24, respectively. TGA results show that Form L has a weight loss of 7.3% during heating to 100°C and may decompose at 330°C or more. DSC results show that Form L has an endothermic peak corresponding to a weight loss of TGA at around 80°C and a crystal transformation exothermic peak at around 158°C. The results of the thermal crystal transformation experiments show that Form L transforms into Form A when heated to 100°C and transforms to Form C when heated to 190°C. NMR results show no change in the structure of the compound, and a solvent peak visible at 3.57 ppm for dioxane, with a roughly estimated mass percentage of about 0.6%. The water content test results show that Form L has a water content of 8.7%, which corresponds to the weight loss of TGA. In summary, Form L is a dihydrate.

The XRPD diffraction peaks for Form L are listed in the table below:

| Index | Angle(°) | d Value (Å) | Rel.Intensity |
|---|---|---|---|
| 1 | 5.72 | 15.45471 | 100.0 |
| 2 | 9.98 | 8.89180 | 7.0 |
| 3 | 11.49 | 7.73176 | 69.5 |
| 4 | 12.56 | 7.08311 | 8.6 |
| 5 | 13.28 | 6.70595 | 4.7 |
| 6 | 14.33 | 6.22402 | 10.9 |
| 7 | 16.24 | 5.51019 | 5.8 |
| 8 | 17.32 | 5.17365 | 25.5 |
| 9 | 18.22 | 4.92775 | 3.7 |
| 10 | 20.78 | 4.34165 | 5.1 |
| 11 | 22.65 | 4.00057 | 4.7 |
| 12 | 23.64 | 3.84187 | 4.6 |
| 13 | 24.30 | 3.74349 | 4.2 |
| 14 | 25.33 | 3.60072 | 11.0 |
| 15 | 26.25 | 3.48383 | 10.2 |
| 16 | 27.12 | 3.37959 | 6.4 |
| 17 | 27.51 | 3.33549 | 7.1 |
| 18 | 28.97 | 3.18112 | 6.8 |
| 19 | 29.70 | 3.10898 | 3.5 |
| 20 | 31.88 | 2.91688 | 4.4 |
| 21 | 32.95 | 2.83242 | 4.3 |
| 22 | 33.28 | 2.80749 | 4.5 |
| 23 | 34.91 | 2.69175 | 4.2 |
| 24 | 35.11 | 2.67875 | 4.8 |
| 25 | 42.01 | 2.30205 | 6.0 |

### Example 14 Preparation of Form M of compound of formula I

To 40 mg of the compound of formula I prepared in Example 1, ethylene glycol dimethyl ether was added until completely dissolved. Chloroform was added dropwise until a solid precipitated, stirred for 3-5 h and centrifuged, and the resulting solid was dried under vacuum overnight at room temperature to give Form M, with a slightly less crystallinity. Form M of the compound of formula I was characterised by XRPD, DSC and TGA, as shown in Figures 25-26, respectively. TGA results show that Form M has a weight loss of 10.6% during heating to 200°C and may decompose at 315°C or more. DSC results show that Form M has a crystal transformation exothermic peak at around 228°C. The results of the thermal crystal transformation experiments show that Form M transforms to Form C when heated to 260°C or more. NMR results show no change in the structure of the compound, a solvent peak visible at 8.32 ppm for chloroform, and solvent peaks visible at 0.85 ppm, 1.99 ppm, 2.06 ppm, and 2.30 ppm for 4-methyl-2-pentanone. Based on the integration results, the ratio of the compound to chloroform is 1 : 0.2, and the ratio of the compound to 4-methyl-2-pentanone is 1 : 0.2.

### Example 15 Preparation of Form N of compound of formula I

To 40 mg of the compound of formula I prepared in Example 1, ethylene glycol dimethyl ether was added until completely dissolved. Acetonitrile was added dropwise until a solid precipitated, stirred for 3-5 h and centrifuged, and the resulting solid was dried under vacuum overnight at room temperature to give Form N. Form N of the compound of formula I was characterised by XRPD, DSC and TGA, as shown in Figures 27-28, respectively. TGA results show that Form N has a weight loss of 5.6% during heating to 150°C and may decompose at 315°C or more. DSC results show that Form N has an endothermic signal corresponding to a weight loss of TGA at around 100-160°C and a crystal transformation exothermic peak at around 204°C. The results of the thermal crystal transformation experiments show that Form N transforms to Form C with a slightly less crystallinity when heated to 235°C or more. NMR results show no change in the structure of the compound, and a solvent peak visible at 2.07 ppm for acetonitrile. Based on the integration results, the roughly estimated mass percentage is about 5.0%, corresponding to the weight loss of TGA. In summary, Form N is a solvate of acetonitrile in a ratio of 1 : 0.6 of the compound to acetonitrile.

The XRPD diffraction peaks for Form N are listed in the table below:

| Index | Angle(°) | d Value (Å) | Rel.Intensity |
|---|---|---|---|
| 1 | 7.65 | 11.58018 | 8.5 |
| 2 | 8.42 | 10.51796 | 100 |
| 3 | 9.08 | 9.75853 | 45.1 |
| 4 | 10.85 | 8.18276 | 7 |
| 5 | 16.02 | 5.58175 | 3.5 |
| 6 | 16.86 | 5.31248 | 15.9 |
| 7 | 17.23 | 5.20194 | 13.1 |
| 8 | 19.07 | 4.71449 | 4 |
| 9 | 20.63 | 4.37293 | 3.5 |
| 10 | 21.79 | 4.14945 | 5 |
| 11 | 22.92 | 3.9556 | 3.6 |
| 12 | 28.99 | 3.17917 | 3.7 |

### Example 16 Preparation of Form O of compound of formula I

To 100 mg of the compound of formula I prepared in Example 1, 0.5 ml of N,N-dimethylformamide was added and heated until completely dissolved. Methanol was added dropwise until a solid precipitated, slowly cooled to room temperature, stirred for 3-5 h and centrifuged, and the resulting solid was dried under vacuum overnight at room temperature to give Form O. Form O of the compound of formula I was characterised by XRPD, DSC and TGA, as shown in Figures 29-30, respectively. TGA results show that Form O has a weight loss of 4.7% during heating to 110°C, and a weight loss of 9.8% at 110-150°C, and may decompose at 315°C or more. DSC results show that Form O has endothermic signals corresponding to weight losses of TGA at 80-110°C and 130-160°C. NMR results show no change in the structure of the compound, and solvent peaks visible at 2.73 ppm, 2.89 ppm, and 7.95 ppm for DMF. Based on the integration results, the roughly estimated mass percentage is about 11.7%. In summary, Form O is a solvate of DMF in a ratio of 1 : 0.8 of the compound to DMF.

The XRPD diffraction peaks for Form O are listed in the table below:

| Index | Angle(°) | d Value (Å) | Rel.Intensity |
|---|---|---|---|
| 1 | 5.49 | 16.10940 | 100.0 |
| 2 | 8.64 | 10.25950 | 57.4 |
| 3 | 10.89 | 8.15390 | 99.8 |
| 4 | 11.45 | 7.75765 | 63.9 |
| 5 | 12.39 | 7.18154 | 22.3 |
| 6 | 13.65 | 6.52785 | 31.2 |
| 7 | 15.81 | 5.65528 | 25.9 |
| 8 | 16.37 | 5.46564 | 41.5 |
| 9 | 17.32 | 5.17365 | 17.5 |
| 10 | 17.83 | 5.03158 | 19.8 |
| 11 | 18.18 | 4.93793 | 20.3 |
| 12 | 18.55 | 4.84298 | 53.4 |
| 13 | 20.10 | 4.48217 | 17.9 |
| 14 | 20.71 | 4.35723 | 27.1 |
| 15 | 21.44 | 4.21388 | 44.4 |
| 16 | 22.14 | 4.08712 | 62.7 |
| 17 | 22.73 | 3.98761 | 56.1 |
| 18 | 22.98 | 3.94611 | 41.2 |
| 19 | 23.25 | 3.90245 | 36.7 |
| 20 | 24.94 | 3.65316 | 31.8 |
| 21 | 25.20 | 3.61888 | 35.7 |
| 22 | 26.17 | 3.49345 | 61.2 |
| 23 | 26.91 | 3.40442 | 37.3 |
| 24 | 27.57 | 3.32898 | 34.4 |
| 25 | 28.56 | 3.22268 | 30.6 |
| 26 | 29.61 | 3.11826 | 27.2 |
| 27 | 30.44 | 3.04054 | 17.9 |
| 28 | 31.32 | 2.96390 | 24.3 |
| 29 | 32.29 | 2.88393 | 21.1 |
| t30 | 33.09 | 2.82209 | 24.6 |

### Example 17 Preparation of Form P of compound of formula I

To 100 mg of the compound of formula I prepared in Example 1, 1 ml of methanol was added and heated to reflux. 1 ml of toluene was added dropwise, slowly cooled to room temperature, stirred for 3-5 h and centrifuged, and the resulting solid was dried under vacuum overnight at room temperature to give Form P. Form P of the compound of formula I was characterised by XRPD, DSC and TGA, as shown in Figures 31-32, respectively. TGA results show that Form P has a weight loss of 8.0% during heating to 150°C and may decompose at 315°C or more. DSC results show that Form P has an endothermic signal corresponding to a weight loss of TGA at around 75-130°C. NMR results show no change in the structure of the compound, solvent peaks visible at 2.30 ppm, 7.18 ppm, 7.24 ppm for toluene, and solvent peaks visible at 3.17 ppm and 4.10 ppm for methanol. Based on the integration results, the roughly estimated methanol mass percentage is about 1.7%. The water content test results show that Form P has a water content of 8.2%, which corresponds to the weight loss of TGA. In summary, Form P is a dihydrate.

The XRPD diffraction peaks for Form P are listed in the table below:

| Index | Angle(°) | d Value (Å) | Rel.Intensity |
|---|---|---|---|
| 1 | 5.76 | 15.35077 | 100.0 |
| 2 | 10.58 | 8.39071 | 15.8 |
| 3 | 10.99 | 8.08266 | 15.0 |
| 4 | 11.55 | 7.69326 | 73.8 |
| 5 | 12.54 | 7.09391 | 9.9 |
| 6 | 14.60 | 6.11050 | 16.7 |
| 7 | 15.40 | 5.80139 | 8.0 |
| 8 | 15.75 | 5.67568 | 9.0 |
| 9 | 17.13 | 5.23056 | 15.2 |
| 10 | 17.38 | 5.15683 | 27.4 |
| 11 | 17.79 | 5.04222 | 10.2 |
| 12 | 18.92 | 4.75180 | 5.6 |
| 13 | 19.29 | 4.66419 | 6.8 |
| 14 | 19.58 | 4.59740 | 11.6 |
| 15 | 20.47 | 4.40470 | 5.8 |
| 16 | 21.25 | 4.25061 | 10.8 |
| 17 | 21.85 | 4.13892 | 10.9 |
| 18 | 22.11 | 4.09394 | 11.3 |
| 19 | 23.12 | 3.92414 | 40.8 |
| 20 | 23.97 | 3.79198 | 6.0 |
| 21 | 24.50 | 3.71560 | 41.2 |
| 22 | 24.77 | 3.67732 | 9.5 |
| 23 | 25.29 | 3.60589 | 8.6 |
| 24 | 25.72 | 3.54992 | 18.3 |
| 25 | 25.91 | 3.52512 | 21.0 |
| 26 | 26.58 | 3.44361 | 25.4 |
| 27 | 27.20 | 3.37067 | 12.5 |
| 28 | 28.27 | 3.25313 | 6.3 |
| 29 | 28.64 | 3.21467 | 7.8 |
| 30 | 29.51 | 3.12760 | 17.1 |
| 31 | 30.66 | 3.02138 | 7.2 |
| 32 | 30.87 | 3.00250 | 8.9 |
| 33 | 31.43 | 2.95403 | 7.7 |
| 34 | 32.93 | 2.83391 | 6.7 |
| 35 | 33.20 | 2.81331 | 10.5 |
| 36 | 33.46 | 2.79451 | 11.8 |
| 37 | 35.17 | 2.67488 | 8.5 |
| 38 | 36.18 | 2.60994 | 5.9 |
| 39 | 36.55 | 2.58719 | 5.5 |
| 40 | 37.75 | 2.51636 | 4.8 |
| 41 | 39.42 | 2.42600 | 6.0 |
| 42 | 40.32 | 2.38110 | 5.0 |
| 43 | 41.46 | 2.32669 | 7.2 |

### Example 18 Preparation of Form Q of compound of formula I

To 100 mg of Form A of the compound of formula I, 1 ml of tetrahydrofuran was added, stirred at room temperature for 3-5 h and centrifuged, and the resulting solid was dried under vacuum at room temperature overnight, to obtain Form Q. Form Q of the compound of formula I was characterised by XRPD, DSC and TGA, as shown in Figures 33-34, respectively. TGA results show that Form Q has a weight loss of 13.2% during heating to 200°C and may decompose at 330°C or more. DSC results show that Form Q has an endothermic signal corresponding to a weight loss of TGA at 190°C or less. NMR results show no change in the structure of the compound, and solvent peaks visible at 1.75 ppm and 3.59 ppm for tetrahydrofuran. Based on the integration results, the ratio of the compound to tetrahydrofuran is 1 : 0.6, and the roughly estimated mass percentage is about 8.4%. In summary, Form Q may be a tetrahydrofuran solvate containing crystal water.

### Example 19 Preparation of Form R of compound of formula I

To 100 mg of the compound of formula I prepared in Example 1, dioxane was added until completely dissolved. Diethyl ether was added dropwise until a solid precipitated, stirred for 3-5 h and centrifuged, and the resulting solid was dried under vacuum overnight at room temperature to give Form R. Form R of the compound of formula I was characterised by XRPD, DSC and TGA, as shown in Figures 35-36, respectively. TGA results show that Form R has a weight loss of 15.6% during heating to 200°C and may decompose at 315°C or more. DSC results show that Form R has an endothermic signal corresponding to a weight loss of TGA at around 185°C. The results of the thermal crystal transformation experiments show that Form R transforms to Form C by desolvation. NMR results show no change in the structure of the compound, a solvent peak visible at 3.57 ppm for dioxane, and solvent peaks visible at 1.09 ppm and 3.38 ppm for diethyl ether. Based on the integration results, the roughly estimated mass percentage is around 14.7%, and the ratio of the compound to diethyl ether is 1 : 0.3, and the roughly estimated mass percentage is around 4.1%. In summary, Form R is a solvate of dioxane in a ratio of 1 : 0.9 of the compound to dioxane.

### Example 20 Preparation of Form S of compound of formula I

To 100 mg of the compound of formula I prepared in Example 1, 0.5 ml of dimethyl sulfoxide was added and heated until completely dissolved, and then cooled to room temperature. Ethanol was added dropwise until a solid precipitated, stirred for 3-5 h and centrifuged, and the resulting solid was dried under vacuum overnight at room temperature to give Form S. Form S of the compound of formula I was characterised by XRPD, DSC and TGA, as shown in Figures 37-38, respectively. TGA results show that Form S has a weight loss of 15.0% during heating to 230°C and may decompose at 315°C or more. DSC results show that Form S has an endothermic signal corresponding to a weight loss of TGA at around 190-230°C. NMR results show no change in the structure of the compound, and a solvent peak visible at 2.54 ppm for DMSO. Based on the integration results, the roughly estimated mass percentage is about 14.2%, corresponding to the weight loss of TGA. In summary, Form S is a solvate of DMSO in a ratio of 1 : 1 of the compound to DMSO.

The XRPD diffraction peaks for Form S are listed in the table below:

| Index | Angle(°) | d Value (Å) | Rel.Intensity |
|---|---|---|---|
| 1 | 6.81 | 12.99347 | 17.7 |
| 2 | 7.74 | 11.43570 | 100.0 |
| 3 | 8.46 | 10.46999 | 64.5 |
| 4 | 10.29 | 8.62581 | 25.9 |
| 5 | 12.37 | 7.19265 | 29.5 |
| 6 | 13.55 | 6.57377 | 30.5 |
| 7 | 15.61 | 5.72389 | 35.9 |
| 8 | 15.85 | 5.64176 | 5.6 |
| 9 | 16.14 | 5.54248 | 6.7 |
| 10 | 16.51 | 5.42183 | 7.2 |
| 11 | 16.88 | 5.30654 | 3.5 |
| 12 | 17.52 | 5.11802 | 9.1 |
| 13 | 18.04 | 4.97391 | 8.6 |
| 14 | 18.65 | 4.81863 | 43.5 |
| 15 | 19.09 | 4.70987 | 26.0 |
| 16 | 20.36 | 4.42885 | 9.3 |
| 17 | 20.59 | 4.38083 | 21.2 |
| 18 | 21.11 | 4.27674 | 7.3 |
| 19 | 21.83 | 4.14243 | 4.4 |
| 20 | 22.40 | 4.04334 | 17.4 |
| 21 | 22.67 | 3.99733 | 5.8 |
| 22 | 23.00 | 3.94295 | 10.5 |
| 23 | 23.23 | 3.90553 | 49.5 |
| 24 | 23.49 | 3.86585 | 37.7 |
| 25 | 23.82 | 3.81528 | 7.3 |
| 26 | 24.07 | 3.77758 | 18.1 |
| 27 | 24.63 | 3.69635 | 12.0 |
| 28 | 24.79 | 3.67461 | 23.7 |
| 29 | 25.06 | 3.63725 | 4.8 |
| 30 | 25.93 | 3.52266 | 21.4 |
| 31 | 26.25 | 3.48383 | 12.0 |
| 32 | 26.96 | 3.39761 | 6.6 |
| 33 | 27.47 | 3.33984 | 11.2 |
| 34 | 28.25 | 3.25518 | 17.8 |
| 35 | 28.87 | 3.19090 | 15.6 |
| 36 | 29.28 | 3.15028 | 5.7 |
| 37 | 29.65 | 3.11454 | 5.3 |
| 38 | 30.50 | 3.03528 | 12.2 |
| 39 | 31.20 | 2.97386 | 6.6 |
| 40 | 31.71 | 2.93129 | 6.3 |
| 41 | 32.37 | 2.87775 | 4.9 |
| 42 | 32.78 | 2.84584 | 4.7 |
| 43 | 33.03 | 2.82651 | 6.6 |
| 44 | 33.26 | 2.80894 | 7.2 |
| 45 | 34.16 | 2.74400 | 5.1 |
| 46 | 34.41 | 2.72631 | 4.4 |
| 47 | 35.59 | 2.64691 | 6.0 |
| 48 | 36.06 | 2.61723 | 5.5 |
| 49 | 36.33 | 2.60030 | 4.8 |
| 50 | 37.73 | 2.51746 | 4.8 |
| 51 | 39.15 | 2.44012 | 10.6 |
| 52 | 39.81 | 2.40620 | 3.8 |
| 53 | 41.31 | 2.33387 | 6.4 |
| 54 | 42.51 | 2.27981 | 5.0 |

### Example 21 Preparation of Form T of compound of formula I

To 100 mg of the compound of formula I prepared in Example 1, 4-methyl-2-pentanone was added until completely dissolved, and volatilised at room temperature until a solid precipitated, and the resulting solid was centrifuged, dried under vacuum overnight at room temperature to give Form T. Form T of the compound of formula I was characterised by XRPD, DSC and TGA, as shown in Figures 39-40, respectively. TGA results show that Form T has a weight loss of 9.7% during heating to 130°C, and a weight loss of 8.1% at 130-200°C, and decomposes at 315°C or more. DSC results show that Form T has an endothermic signal corresponding to a weight loss of TGA at around 100-170°C. NMR results show no change in the structure of the compound, and solvent peaks visible at 0.85 ppm, 1.99 ppm, 2.06 ppm, and 2.30 ppm for 4-methyl-2-pentanone. Based on the integration results, the roughly estimated mass percentage is 18.0%, corresponding to the weight loss of TGA. In summary, Form T is a solvate of 4-methyl-2-pentanone in a ratio of 1 : 1 of the compound to 4-methyl-2-pentanone.

The XRPD diffraction peaks for Form T are listed in the table below:

| Index | Angle(°) | d Value (Å) | Rel.Intensity |
|---|---|---|---|
| 1 | 6.81 | 12.99347 | 47.9 |
| 2 | 6.98 | 12.66961 | 100.0 |
| 3 | 7.78 | 11.37892 | 87.3 |
| 4 | 9.76 | 9.08455 | 31.5 |
| 5 | 11.82 | 7.51865 | 48.2 |
| 6 | 13.22 | 6.73499 | 13.9 |
| 7 | 13.65 | 6.52785 | 43.8 |
| 8 | 15.07 | 5.92554 | 16.9 |
| 9 | 15.36 | 5.81572 | 41.1 |
| 10 | 16.59 | 5.39713 | 11.9 |
| 11 | 17.42 | 5.14568 | 24.7 |
| 12 | 17.77 | 5.04755 | 18.5 |
| 13 | 18.26 | 4.91762 | 36.1 |
| 14 | 18.51 | 4.85279 | 28.7 |
| 15 | 19.66 | 4.57993 | 5.9 |
| 16 | 20.41 | 4.41674 | 16.3 |
| 17 | 20.69 | 4.36114 | 13.0 |
| 18 | 21.17 | 4.26550 | 14.9 |
| 19 | 21.46 | 4.21024 | 17.9 |
| 20 | 21.78 | 4.15298 | 26.6 |
| 21 | 22.22 | 4.07354 | 30.7 |
| 22 | 22.73 | 3.98761 | 15.3 |
| 23 | 23.08 | 3.93039 | 20.4 |
| 24 | 23.54 | 3.85682 | 9.8 |
| 25 | 23.82 | 3.81528 | 28.4 |
| 26 | 24.03 | 3.78333 | 11.8 |
| 27 | 25.04 | 3.63989 | 34.3 |
| 28 | 25.31 | 3.60330 | 19.0 |
| 29 | 25.70 | 3.55243 | 16.0 |
| 30 | 25.91 | 3.52512 | 14.7 |
| 31 | 26.13 | 3.49828 | 7.8 |
| 32 | 26.67 | 3.43198 | 16.9 |
| 33 | 26.83 | 3.41355 | 17.9 |
| 34 | 26.98 | 3.39535 | 16.4 |
| 35 | 27.33 | 3.35517 | 7.3 |
| 36 | 27.72 | 3.31178 | 10.6 |
| 37 | 27.96 | 3.28635 | 13.6 |
| 38 | 28.52 | 3.22670 | 24.5 |
| 39 | 29.02 | 3.17529 | 7.1 |
| 40 | 29.41 | 3.13700 | 6.9 |
| 41 | 30.05 | 3.07612 | 16.5 |
| 42 | 30.91 | 2.99909 | 6.5 |
| 43 | 31.26 | 2.96887 | 11.6 |
| 44 | 31.90 | 2.91530 | 12.1 |
| 45 | 32.23 | 2.88858 | 8.4 |
| 46 | 33.18 | 2.81477 | 8.4 |
| 47 | 34.27 | 2.73580 | 8.1 |
| 48 | 34.74 | 2.70359 | 6.6 |
| 49 | 35.32 | 2.66462 | 10.1 |
| 50 | 35.65 | 2.64316 | 6.5 |
| 51 | 36.06 | 2.61723 | 7.4 |
| 52 | 36.55 | 2.58719 | 9.1 |
| 53 | 39.97 | 2.39840 | 7.0 |
| 54 | 40.92 | 2.35208 | 7.9 |
| 55 | 41.85 | 2.30901 | 6.8 |
| 56 | 44.11 | 2.21351 | 7.4 |

### Example 22 Preparation of Form U of compound of formula I

To 100 mg of the compound of formula I prepared in Example 1, 1 ml of methanol was added and heated to reflux. 1 ml of acetonitrile was added dropwise, slowly cooled to room temperature, stirred for 3-5 h and centrifuged, and the resulting solid was dried under vacuum overnight at room temperature to give Form U. Form U of the compound of formula I was characterised by XRPD, DSC and TGA, as shown in Figures 41-42, respectively. TGA results show that Form U has a weight loss of 8.1% during heating to 200°C and decomposes at 250°C or more. DSC results show that Form U has an endothermic signal corresponding to a weight loss of TGA at around 80-150°C and a crystal transformation exothermic signal at 155-200°C. During the experiment, it was found that Form U transformed to Form L by leaving to stand at room temperature for 3 days. The results of the thermal crystal transformation experiments show that Form U transforms to Form C when heated to 250°C. NMR results show no change in the structure of the compound, and a solvent peak visible at 2.07 ppm for acetonitrile. Based on the integration results, the roughly estimated mass percentage is about 5.2%. In summary, Form U is a solvate of acetonitrile in a ratio of 1 : 1 of the compound to acetonitrile.

### Example 23 Preparation of Form V of compound of formula I

To 100 mg of the compound of formula I prepared in Example 1, 1 ml of chloroform and 1 ml of ethanol were added and stirred at room temperature for 10-15 hours and centrifuged, and the resulting solid was dried under vacuum overnight at room temperature to give Form V. Form V of the compound of formula I was characterised by XRPD, DSC and TGA, as shown in Figures 43-44, respectively. TGA results show that Form V has a weight loss of 3.4% during heating to 200°C and decomposes at 315°C or more. DSC results show that Form V has no significant heat flow signal at 300°C or less. The results of the thermal crystal transformation experiments show that Form V is still Form V when heated to 150°C, and the TGA results of the samples obtained by thermal crystal transformation show a 1.6% weight loss when heated to 150°C. NMR results show no change in the structure of the compound, and a solvent peak visible at 8.32 ppm for chloroform. Based on the integration results, the ratio of the compound to chloroform is 1 : 0.1. In summary, Form V is an anhydrate.

The XRPD diffraction peaks for Form V are listed in the table below:

| Index | Angle(°) | d Value (Å) | Rel.Intensity |
|---|---|---|---|
| 1 | 5.27 | 16.76049 | 27.0 |
| 2 | 6.60 | 13.41269 | 23.0 |
| 3 | 7.41 | 11.94244 | 100.0 |
| 4 | 11.05 | 8.04052 | 78.1 |
| 5 | 11.40 | 7.79681 | 14.2 |
| 6 | 11.73 | 7.58007 | 11.6 |
| 7 | 12.02 | 7.39881 | 13.5 |
| 8 | 13.71 | 6.50061 | 12.5 |
| 9 | 14.16 | 6.29933 | 20.6 |
| 10 | 14.35 | 6.21577 | 10.0 |
| 11 | 14.88 | 6.00118 | 9.9 |
| 12 | 15.59 | 5.73085 | 24.1 |
| 13 | 15.81 | 5.65528 | 37.2 |
| 14 | 16.45 | 5.44052 | 12.8 |
| 15 | 16.97 | 5.27704 | 51.3 |
| 16 | 17.56 | 5.10705 | 10.7 |
| 17 | 18.57 | 4.83809 | 12.8 |
| 18 | 18.78 | 4.78497 | 13.4 |
| 19 | 19.33 | 4.65517 | 13.6 |
| 20 | 19.73 | 4.56260 | 23.0 |
| 21 | 19.91 | 4.52412 | 12.8 |
| 22 | 21.33 | 4.23584 | 19.8 |
| 23 | 22.53 | 4.02019 | 37.1 |
| 24 | 22.82 | 3.97154 | 17.1 |
| 25 | 23.29 | 3.89630 | 47.6 |
| 26 | 23.78 | 3.82116 | 28.1 |
| 27 | 24.55 | 3.70732 | 14.3 |
| 28 | 25.39 | 3.59300 | 33.9 |
| 29 | 26.38 | 3.46714 | 11.3 |
| 30 | 27.63 | 3.32251 | 39.7 |
| 31 | 28.23 | 3.25724 | 19.0 |
| 32 | 28.56 | 3.22268 | 12.6 |
| 33 | 28.95 | 3.18307 | 13.4 |
| 34 | 29.41 | 3.13700 | 9.4 |
| 35 | 30.48 | 3.03703 | 8.7 |
| 36 | 31.14 | 2.97886 | 9.2 |
| 37 | 32.19 | 2.89169 | 13.9 |
| 38 | 33.03 | 2.82651 | 9.6 |
| 39 | 33.75 | 2.77319 | 18.9 |
| 40 | 35.90 | 2.62703 | 9.6 |
| 41 | 37.85 | 2.51086 | 8.8 |
| 42 | 39.31 | 2.43203 | 7.1 |
| 43 | 39.85 | 2.40424 | 7.0 |
| 44 | 41.95 | 2.30465 | 7.1 |

### Example 24 Preparation of Form W of compound of formula I

To 20 mg of the compound of formula I prepared in Example 1, ethylene glycol monomethyl ether was added until completely dissolved, and placed into diethyl ether for vapor diffusion, and the resulting solid was dried under vacuum overnight at room temperature to give Form W. Form W of the compound of formula I was characterised by XRPD, as shown in Figure 45. NMR results show no change in the structure of the compound, solvent peaks visible at 1.09 ppm, and 3.38 ppm for diethyl ether, and solvent peaks visible at 3.24 ppm, 3.48 ppm and 4.56 ppm for ethylene glycol monomethyl ether. Based on the integration results, the ratio of the compound to diethyl ether is 1 : 0.3, the roughly estimated mass percentage is about 3.7% and the roughly estimated mass percentage is about 10.1%. In summary, Form W is a solvate of ethylene glycol monomethyl ether, with a ratio of 1 : 0.7 of the compound to ethylene glycol monomethyl ether.

The XRPD patterns of Form F, Form Q, Form R, Form S, Form U and Form W are similar, and presumably the six forms are heteroisomorphic solvates.

### Example 25 Preparation of Form X of compound of formula I

To 200 mg of Form E of the compound of formula I, 1 ml of toluene was added, stirred at 50-60°C for 10-15 h and filtered, and the resulting solid was dried under vacuum at room temperature overnight, to obtain Form X. Form X of the compound of formula I was characterised by XRPD, as shown in Figure 46.

### Example 26 Preparation of Form Y of compound of formula I

To 200 mg of Form G of the compound of formula I, 1 ml of acetonitrile was added, stirred at 50-60°C for 10-15 h and filtered, and the resulting solid was dried under vacuum at room temperature overnight, to obtain Form Y. Form Y of the compound of formula I was characterised by XRPD, as shown in Figure 47.

### Example 27 Preparation of Form Z of compound of formula I

To 200 mg of the compound of formula I prepared in Example 1, 1 ml of water and 1 ml of N,N-dimethylformamide were added, stirred at room temperature for 10-15 hours and filtered, and the resulting solid was dried under vacuum overnight at room temperature to give Form Z. Form Z of the compound of formula I was characterised by XRD, DSC and TGA, as shown in Figures 48-49, respectively. TGA results show that Form Z has a weight loss of 0.5% during heating to 100°C, and a weight loss of 13.3% at 100-200°C, and decomposes at 330°C or more. DSC results show that Form Z has endothermic signals corresponding to weight losses of TGA at 50-80°C and 110-180°C. NMR results show no change in the structure of the compound, and solvent peaks visible at 2.73 ppm, 2.89 ppm, and 7.95 ppm for DMF. Based on the integration results, the roughly estimated mass percentage is 14.0%, corresponding to the weight loss of TGA. In summary, Form Z is a solvate of DMF in a ratio of about 1 : 0.9 of the compound to DMF.

The XRPD diffraction peaks for Form Z are listed in the table below:

| Index | Angle(°) | d Value (Å) | Rel.Intensity |
|---|---|---|---|
| 1 | 5.68 | 15.56011 | 19.5 |
| 2 | 8.34 | 10.61526 | 39.6 |
| 3 | 10.17 | 8.72364 | 23.3 |
| 4 | 11.40 | 7.79681 | 25.4 |
| 5 | 12.00 | 7.41061 | 12.7 |
| 6 | 13.51 | 6.59232 | 24.9 |
| 7 | 14.84 | 6.01655 | 19.9 |
| 8 | 15.56 | 5.74481 | 15.3 |
| 9 | 16.02 | 5.58175 | 14.5 |
| 10 | 17.44 | 5.14012 | 14.2 |
| 11 | 18.28 | 4.91256 | 20.1 |
| 12 | 18.76 | 4.78975 | 18.4 |
| 13 | 19.29 | 4.66419 | 14.4 |
| 14 | 19.46 | 4.62387 | 12.2 |
| 15 | 20.16 | 4.46974 | 26.3 |
| 16 | 20.45 | 4.40871 | 21.8 |
| 17 | 20.73 | 4.35332 | 22.7 |
| 18 | 21.15 | 4.26924 | 100.0 |
| 19 | 21.62 | 4.18140 | 17.0 |
| 20 | 22.84 | 3.96834 | 14.4 |
| 21 | 23.17 | 3.91481 | 6.9 |
| 22 | 23.56 | 3.85382 | 6.3 |
| 23 | 24.71 | 3.68544 | 25.4 |
| 24 | 25.10 | 3.63198 | 13.4 |
| 25 | 25.31 | 3.60330 | 11.8 |
| 26 | 25.99 | 3.51531 | 20.9 |
| 27 | 26.50 | 3.45298 | 29.0 |
| 28 | 26.79 | 3.41814 | 22.9 |
| 29 | 27.10 | 3.38184 | 10.6 |
| 30 | 27.59 | 3.32682 | 8.8 |
| 31 | 28.75 | 3.20273 | 16.9 |
| 32 | 29.74 | 3.10529 | 8.3 |
| 33 | 30.04 | 3.07792 | 21.2 |
| 34 | 30.48 | 3.03703 | 5.5 |
| 35 | 30.99 | 2.99231 | 10.2 |
| 36 | 32.06 | 2.90265 | 15.6 |
| 37 | 32.48 | 2.86855 | 6.1 |
| 38 | 32.83 | 2.84135 | 5.9 |
| 39 | 33.13 | 2.81916 | 6.0 |
| 40 | 33.77 | 2.77178 | 7.1 |
| 41 | 34.56 | 2.71556 | 7.2 |
| 42 | 35.48 | 2.65447 | 9.5 |
| 43 | 35.89 | 2.62826 | 7.0 |
| 44 | 37.38 | 2.53753 | 6.0 |
| 45 | 38.86 | 2.45550 | 6.2 |
| 46 | 40.32 | 2.38110 | 5.5 |
| 47 | 42.92 | 2.26229 | 4.5 |
| 48 | 44.32 | 2.20504 | 4.1 |

### Thermal Crystal Transformation test

The different crystal forms, as raw materials, were heated to the target temperature on a hot table and held for 1 min, and cooled to room temperature to obtain solids for the XRPD test. The results are shown in the table below.

### Thermal crystal transformation results

| Experiment No. | Raw crystal form | Target temperature (°C) | Heating rate | Temperature-holding time | Results |
|---|---|---|---|---|---|
| 1 | Form A | 150 | 10°C/min | 1 min | Form A |
| 2 | Form A | 300 | 10°C/min | 1 min | Form C |
| 3 | Form E | 180 | 10°C/min | 1 min | Form C |
| 4 | Form F | 230 | 10°C/min | 1 min | Form C |
| 5 | Form J | 160 | 10°C/min | 1 min | Form C |
| 6 | Form K | 260 | 10°C/min | 1 min | Form C |
| 7 | Form L | 100 | 10°C/min | 1 min | Form A |
| 8 | Form L | 190 | 10°C/min | 1 min | Form C |
| 9 | Form N | 235 | 10°C/min | 1 min | Form C |
| 10 | Form R | 200 | 10°C/min | 1 min | Form C |
| 11 | Form M | 260 | 10°C/min | 1 min | Form C |
| 12 | Form U | 250 | 10°C/min | 1 min | Form C |

The results show that many forms are transformed into Form C by heating, which is a more stable form at elevated temperatures and very useful as an active ingredient in pharmaceutical formulations. The active ingredient is not easily transformed by heat during the preparation process, which in turn affects its parameters such as bioavailability, and is stable even when stored at temperatures above room temperature.

Dynamic Moisture Sorption and Desorption Analysis The dynamic moisture sorption and desorption analysis was determined using DVS Intrinsic (SMS, UK). The test was carried out in a gradient mode with a humidity change of 50%-95%-0%-50%, and a humidity change of 10% per gradient in the range of 0% to 90%, and the gradient endpoint was determined in dm/dt, with the gradient endpoint being dm/dt less than 0.002% and held for 10 minutes. After the test was complete, XRPD analysis of the sample was carried out to confirm whether the solid morphology has changed.

The DVS results are shown in Figure 50. The DVS results show that Form C has a weight gain of 0.20% at 95% humidity and a weight loss of 0.13% at 0% humidity, indicating that Form C has almost no hygroscopicity, and the XRPD results show that the samples after the DVS test have no change in crystal form. As a crystal form free of water, Form C does not change to a crystal form containing water under high moisture conditions by wet adsorption, which is very beneficial for pharmaceutical preservation and preparation processes.

### Stability study

| **Related Substance Analysis Methods (HPLC)** | | | | |
|---|---|---|---|---|
| Instrument | Model | High-performance liquid chromatograph, equipped with VWD or DAD detector | | |
| Parameters and methods | Chromatographic column | Agilent Eclipse XDB-Cis 4.6 mm × 250 mm, 5 µm or equivalent | | |
| | Trap column | Ghost-Sniper Column 4.6 mm × 50 mm or equivalent | | |
| | Column temperature | 30°C | | |
| | Flow rate | 1.0 ml/min | | |
| | Detection wavelength | 210 nm | | |
| | Injection volume | 10 µl | | |
| | Operation time | 60 min | | |
| | Mobile Phase A | 0.01 mol/L sodium dihydrogen phosphate solution (pH adjusted to 3.0 with phosphoric acid) | | |
| | Mobile phase B | Acetonitrile | | |
| | | Time (min) | Mobile phase A (%) | Mobile phase B (%) |
| | | 0 | 75 | 25 |
| | | 5 | 70 | 30 |
| | | 10 | 60 | 40 |
| | Operating gradient | 25 | 58 | 42 |
| | | 40 | 35 | 65 |
| | | 52 | 35 | 65 |
| | | 53 | 75 | 25 |
| | | 60 | 75 | 25 |

### Stability test 1

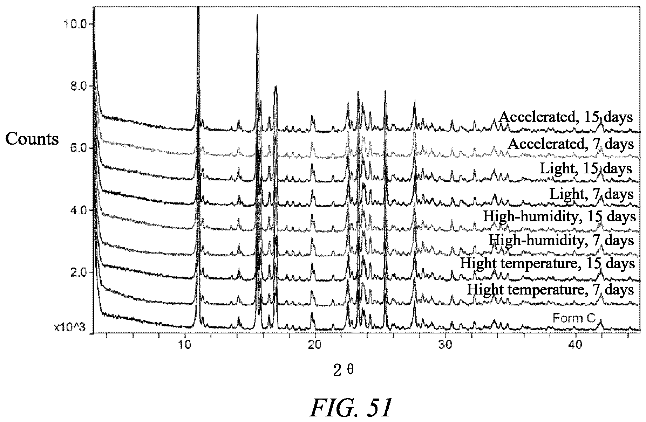

Around 20 mg of samples were weighed into weighing bottles, stored under high temperature (60°C), high humidity (25°C, 92.5% RH), light (25°C, 4500 Lux), accelerated (40°C, 75% RH) conditions, respectively, and samples were collected at days 7 and 15 for XRPD characterization. The XRPD results in FIG. 51 show that Form C under each of the high temperature, high humidity, light, and accelerated conditions is stable for 15 days, with no change in crystal form.

### Stability test 2

The Form C sample was measured using the HPLC method at 40°C ± 2°C, 75% RH ± 5% RH at months 0, 1, 2, 3, and 6 using a double layer pharmaceutical low density polyethylene bag as an inner wrap and a polyester/aluminium/polyethylene pharmaceutical composite bag as an outer wrap. The results are shown in the table below.

| **Examination item** | | **Time (months)** | | | | |
|---|---|---|---|---|---|---|
| | | **0** | **1** | **2** | **3** | **6** |
| Related Substances (%) | Maximum individual impurity | 0.05 | 0.05 | 0.05 | 0.05 | 0.05 |
| | Total impurity | 0.26 | 0.25 | 0.26 | 0.26 | 0.28 |
| Crystal form | | Form C | Form C | Form C | Form C | Form C |

The results show that: Form C has good stability within 6 months at 40°C ± 2°C, 75% RH ± 5% RH, with no significant increase in the related substances.

### Stability test 3

The Form C sample was measured using the HPLC method at 5°C ± 3°C at months 0, 3, 6, 9, and 12 using a double layer pharmaceutical low density polyethylene bag as an inner wrap and a polyester/aluminium/polyethylene pharmaceutical composite bag as an outer wrap. The results are shown in the table below.

| **Examination item** | | **Time (months)** | | | | |
|---|---|---|---|---|---|---|
| | | **0** | **3** | **6** | **9** | **12** |
| Related Substances (%) | Maximum individual impurity | 0.05 | 0.05 | 0.05 | 0.05 | 0.05 |
| | Total impurity | 0.26 | 0.27 | 0.26 | 0.26 | 0.26 |
| Crystal form | | Form C | Form C | Form C | Form C | Form C |

The results show that: Form C has good stability within 12 months at 5°C ± 3°C, with no significant increase in the related substances.

### Stability test 4

The Form C sample was measured using the HPLC method at 25°C ± 2°C, 60% RH ± 5% RH at months 0, 3, 6, 9, and 12 using a double layer pharmaceutical low density polyethylene bag as an inner wrap and a polyester/aluminium/polyethylene pharmaceutical composite bag as an outer wrap. The results are shown in the table below.

| **Examination item** | | **Time (months)** | | | | |
|---|---|---|---|---|---|---|
| | | **0** | **3** | **6** | **9** | **12** |
| Related Substances (%) | Maximum individual impurity | 0.05 | 0.05 | 0.05 | 0.05 | 0.05 |
| | Total impurity | 0.26 | 0.27 | 0.26 | 0.26 | 0.27 |
| Crystal form | | Form C | Form C | Form C | Form C | Form C |

The results show that: Form C has good stability within 12 months at 25°C ± 2°C, 60% RH ± 5% RH, with no significant increase in the related substances.

### Stability test 5

The Form C sample was measured using the HPLC method at 30°C ± 2°C, 65% RH ± 5% RH at months 0, 3, 6, 9, and 12 using a double layer pharmaceutical low density polyethylene bag as an inner wrap and a polyester/aluminium/polyethylene pharmaceutical composite bag as an outer wrap. The results are shown in the table below.

| **Examination item** | | **Time (months)** | | | | |
|---|---|---|---|---|---|---|
| | | **0** | **3** | **6** | **9** | **12** |
| Related Substances (%) | Maximum individual impurity | 0.05 | 0.05 | 0.05 | 0.05 | 0.05 |
| | Total impurity | 0.26 | 0.26 | 0.27 | 0.25 | 0.26 |
| Crystal form | | Form C | Form C | Form C | Form C | Form C |

The results show that: Form C has good stability within 12 months at 30°C ± 2°C, 65% RH ± 5% RH, with no significant increase in the related substances.

## Claims

1. A polymorph of a compound of formula I: 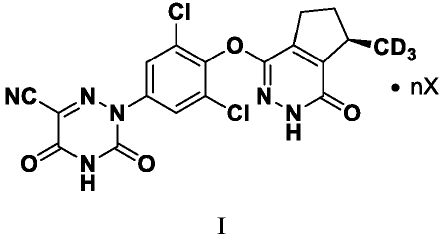 wherein n is 0 to 2 and X is H₂O.

2. The polymorph according to claim 1, **characterised in that** n is 2 and the polymorph is of Form A having an X-ray powder diffraction pattern with characteristic diffraction peaks at the following 2θ positions using Cu-Kα radiation: 5.68° ± 0.2°, 11.44° ± 0.2°, 17.19° ± 0.2°, 24.94° ± 0.2°, 26.40° ± 0.2°, 29.47° ± 0.2°.

3. The polymorph according to claim 2, further having an X-ray powder diffraction pattern with characteristic diffraction peaks at the following 2θ positions: 12.68° ± 0.2°, 19.44° ± 0.2°, 19.79° ± 0.2°, 23.27° ± 0.2°.

4. The polymorph according to claim 2 or 3, having an X-ray powder diffraction pattern substantially as shown in Figure 1.

5. The polymorph according to claim 2 or 3, the Form A has a DSC and/or TGA diagram of Figure 2.

6. The polymorph according to claim 1, **characterised in that** n is 0 and the polymorph is of Form C having an X-ray powder diffraction pattern with characteristic diffraction peaks at the following 2θ positions using Cu-Kα radiation: 11.03° ± 0.2°, 15.79° ± 0.2°, 16.97° ± 0.2°, 23.27° ± 0.2°, 23.76° ± 0.2°, 27.61° ± 0.2°.

7. The polymorph according to claim 6, further having an X-ray powder diffraction pattern with characteristic diffraction peaks at the following 2θ positions: 14.12° ± 0.2°, 19.73° ± 0.2°, 22.51° ± 0.2°, 25.37° ± 0.2°, 28.21° ± 0.2°, 33.73° ± 0.2°.

8. The polymorph according to claim 6 or 7, having an X-ray powder diffraction pattern substantially as shown in Figure 5.

9. The polymorph according to claim 6 or 7, the Form C has a DSC and/or TGA diagram of Figure 6.

10. The polymorph according to claim 1, **characterised in that** n is 2 and the polymorph is of Form L having an X-ray powder diffraction pattern with characteristic diffraction peaks at the following 2θ positions using Cu-Kα radiation: 5.72° ± 0.2°, 11.49° ± 0.2°, 12.56° ± 0.2°, 14.33° ± 0.2°, 17.32° ± 0.2°, 25.33° ± 0.2°, 26.25° ± 0.2°, 27.51° ± 0.2°.

11. The polymorph according to claim 10, having an X-ray powder diffraction pattern substantially as shown in Figure 23.

12. The polymorph according to claim 10, the Form L has a DSC and/or TGA diagram of Figure 24.

13. The polymorph according to claim 1, **characterised in that** n is 2 and the polymorph is of Form P having an X-ray powder diffraction pattern with characteristic diffraction peaks at the following 2θ positions using Cu-Kα radiation: 5.76° ± 0.2°, 11.55° ± 0.2°, 17.38° ± 0.2°, 23.12° ± 0.2°, 24.50° ± 0.2°, 26.58° ± 0.2°.

14. The polymorph according to claim 13, further having an X-ray powder diffraction pattern with characteristic diffraction peaks at the following 2θ positions: 14.60° ± 0.2°, 17.13° ± 0.2°, 25.91° ± 0.2°, 29.51° ± 0.2°.

15. The polymorph according to claim 13 or 14, having an X-ray powder diffraction pattern substantially as shown in Figure 31.

16. The polymorph according to claim 13 or 14, the Form P has a DSC and/or TGA diagram of Figure 32.

17. The polymorph according to claim 1, **characterised in that** n is 0 and the polymorph is of Form V having an X-ray powder diffraction pattern with characteristic diffraction peaks at the following 2θ positions using Cu-Kα radiation: 7.41° ± 0.2°, 11.05° ± 0.2°, 15.81° ± 0.2°, 16.97° ± 0.2°, 22.53° ± 0.2°, 23.29° ± 0.2°, 27.63° ± 0.2°.

18. The polymorph according to claim 17, further having an X-ray powder diffraction pattern with characteristic diffraction peaks at the following 2θ positions: 14.16° ± 0.2°, 15.59° ± 0.2°, 19.73° ± 0.2°, 21.33° ± 0.2°, 23.78° ± 0.2°, 25.39° ± 0.2°, 33.75° ± 0.2°.

19. The polymorph according to claim 17 or 18, having an X-ray powder diffraction pattern substantially as shown in Figure 43.

20. The polymorph according to claim 17 or 18, the Form V has a DSC and/or TGA diagram of Figure 44.

21. A pharmaceutical composition **characterised by** comprising a therapeutically effective amount of a polymorph according to any one of claims 1 to 20, and a pharmaceutically acceptable carrier and/or excipient.

22. Use of a polymorph according to any one of claims 1 to 20 or a pharmaceutical composition according to claim 21 in the manufacture of a medicament for the treatment of primary hypercholesterolemia.
